# EUROPÄISCHE PATENTANMELDUNG

(11) **EP 2 918 270 A1**
(43) Veröffentlichungstag der Anmeldung: **16.09.2015**
(21) Anmeldenummer: 14159306.1
(22) Anmeldetag: 12.03.2014
(51) Int. Cl.: A61K 31/36

(54) **Aromatische Alkensäurederivate zur Appetithemmung und Stimmungsaufhellung**

(71) Anmelder: Symrise AG, 37603 Holzminden (DE)
(72) Erfinder: Ley, Jakob, 37603 Holzminden (DE); Reichelt, Katharina, 37603 Holzminden (DE); Obst, Katja, 37603 Holzminden (DE); Somoza, Veronika, 3400 Weidling (AT); Rohm, Barbara, 1090 Wien (AT)
(74) Vertreter: Fabry, Bernd

(57) **Zusammenfassung**

Vorgeschlagen werden primär aromatische Alkensäurederivate der Formel I, zur Anwendung in einem therapeutischen Verfahren als (a) Mittel zur Reduzierung des Appetits und/oder (b) Mittel zur Vermittlung eines Gefühls von Sättigung und/oder als (c) Mittel zur Reduzierung der Energieaufnahme und/oder als (d) Stimmungsaufheller, sowie die nichttherapeutische Verwendung der aromatischen Alkensäurederivate nach Formel I als (a) Mittel zur Reduzierung des Appetits und/oder (b) Mittel zur Vermittlung eines Gefühls von Sättigung und/oder als (c) Mittel zur Reduzierung der Energieaufnahme und/oder als (c) Stimmungsaufheller.

## Beschreibung

### GEBIET DER ERFINDUNG

Die Erfindung betrifft primär aromatische Alkensäurederivate der Formel I, zur Anwendung in einem therapeutischen Verfahren als (a) Mittel zur Reduzierung des Appetits und/oder (b) Mittel zur Vermittlung eines Gefühls von Sättigung und/oder als (c) Mittel zur Reduzierung der Energieaufnahme und/oder als (d) Stimmungsaufheller, sowie die nicht-therapeutische Verwendung der aromatischen Alkensäurederivate nach Formel I als (a) Mittel zur Reduzierung des Appetits und/oder (b) Mittel zur Vermittlung eines Gefühls von Sättigung und/oder als (c) Mittel zur Reduzierung der Energieaufnahme und/oder als (c) Stimmungsaufheller.

Die Erfindung betrifft ferner ein Verfahren zur (a) Reduzierung des Appetits und/oder (b) Vermittlung eines Gefühls von Sättigung und/oder (c) Reduzierung der Energieaufnahme und/oder (d) Aufhellung der Stimmung. Ferner umfasst die Erfindung Zubereitungen zur Anwendung in bestimmten therapeutischen Verfahren, umfassend mindestens ein aromatisches Alkensäurederivat der Formel I und ein oder mehrerer weitere Stoffe, als (a) Mittel zur Reduzierung des Appetits und/oder (b) Mittel zur Vermittlung eines Gefühls von Sättigung und/oder (c) Reduzierung der Energieaufnahme und/oder (d) Stimmungsaufheller. Die Erfindung betrifft auch die nicht-therapeutische Verwendung entsprechender Zubereitungen. Schließlich betrifft die Erfindung auch aromatische Alkensäurederivate der Formel I umfassende oral konsumierbare Produkte (insbesondere Lebensmittel, Futtermittel und Arzneimittel), wobei das/die aromatische(n) Alkensäurederivat(e) in einer (a) den Appetit reduzierenden und/oder (b) ein Gefühl von Sättigung bewirkenden und/oder (c) die Energieaufnahme reduzierende und/oder (d) die Stimmung aufhellenden, jedoch geringen Konzentration vorliegt.

### STAND DER TECHNIK

Das durch mangelnde Bewegung und/oder zu hohe Nahrungsaufnahme verursachte häufige Auftreten von andauerndem Übergewicht kann zu chronischen Krankheitsbildern wie Fettleibigkeit, Insulinresistenz, Lipidstoffwechselstörungen und/oder Bluthochdruck, deren schwerwiegenden Folgeerkrankungen Diabetes Typ II, Arteriosklerose, Herz- oder Hirninfarkt und damit letztendlich zum verfrühten Tod führen. Insbesondere ein hoher Gehalt an leicht verstoffwechselbaren Kohlenhydraten, Proteinen und vor allem Fetten in der Nahrung führt zur Bildung von Fettdepots und kann schließlich zu den genannten Problemen stark beitragen. Um die Aufnahme dieser hedonisch oft sehr bevorzugten Nahrungsmittelbestandteile, vor allem der Fette und süßen Kohlenhydrate (Zucker), zu begrenzen, werden oft deren Gehalte in kalorienreduzierten Lebensmitteln, den sogenannten "Light-Produkten", stark gesenkt und durch Ersatzstoffe ( wie beispielsweise Verdicker für Fette, nicht-kalorische Süßstoffe statt Zucker) ausgetauscht.

Beim Konsum von "Light-Produkten" kann es bei einem Produkt, dass aufgrund geschickter Formulierung einen vergleichbaren hedonischen Wert wie das hoch-energetische Originalprodukt besitzt, oft vorkommen, dass es in größeren Mengen konsumiert wird und es dann im ungünstigsten Fall sogar zu einer erhöhten Aufnahme kalorisch relevanter Nahrungsmittelbestandteile kommt und somit das Ziel verfehlt wird, die aufgenommene Kalorienmenge abzusenken.

Um einer erhöhten Aufnahme kalorisch relevanter Nahrungsmittelbestandteile entgegenzuwirken, besteht schon länger der Wunsch, Nahrungsbestandteile, insbesondere als sicher bewertete, bereits zugelassene und allgemein akzeptierte Aromastoffe zu finden, die das Hungergefühl und den natürlichen Appetit reduzieren und/oder das Sättigungsgefühl entsprechend erhöhen können. Es ist bekannt, dass durch Erhöhung der Serotonin-Ausschüttung in bestimmten Hirnarealen bei gleichzeitiger Exposition mit Nährstoffen durch die aufgenommenen oral konsumierbaren Produkte (insbesondere Lebensmittel) sowie durch Induktion von Leptin-Rezeptor - und Serotonin-Rezeptor-Proteinen der Appetit negativ und die Sättigung positiv beeinflusst werden können.

Neben Auswirkungen auf den Appetit und auf die Sättigung, kann auch die Stimmung des Konsumenten positiv beeinflusst werden. So sind bereits Lebensmittel bekannt, die Dopamin und Serotonin umfassen und eine stimmungsaufhellende Wirkung besitzen. Sofern Serotonin in ausreichenden Mengen im Hirn vorhanden ist, vermittelt es eine positive Stimmung, eine gute Konzentrationsfähigkeit und Optimismus. Niedrige Serotoninspiegel können hingegen zu Reizbarkeit, Schlafstörungen, mangelnder Konzentrationsfähigkeit und Depressionen führen.

Eine Aufnahme von Serotonin und Dopamin über die Nahrung erhöht die Serotonin- bzw. Dopaminkonzentration im Blut und steht mit einiger Wahrscheinlichkeit auch für Wechselwirkungen mit Rezeptoren im Gehirn zur Verfügung. Um die Serotonin- bzw. Dopaminkonzentration im Gehirn zu erhöhen, ist es vorteilhaft, die Ausschüttung von Serotonin bzw. Dopamin im Hirn zu stimulieren.

Die Aufgabe der vorliegenden Erfindung hat somit darin bestanden, Verbindungen und Stoffe zu finden, die einen Beitrag zur Reduzierung des Appetits und/oder zur Vermittlung eines Gefühls von Sättigung und/oder zur Reduzierung der Energieaufnahme und/oder als Stimmungsaufheller leisten können, um die Körpergesundheit positiv zu beeinflussen. Ferner war es Aufgabe der vorliegenden Erfindung, dass diese Stoffe und Verbindungen nicht toxisch oder nur beschränkt einsetzbar sind, so dass sie im Lebensmittelbereich eingesetzt werden können. Vor allem sollten die Stoffe und Verbindungen bereits in geringe Mengen wirken und möglichst keine unangenehmen sensorischen Noten oder einen Nebengeschmack in Produkten aufweisen.

### BESCHREIBUNG DER ERFINDUNG

Diese Aufgabe wird gelöst durch aromatische Alkensäurederivate der Formel I 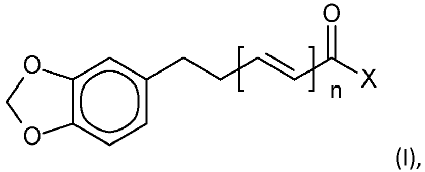 zur Anwendung in einem therapeutischen Verfahren
(i) zur Reduzierung des Appetits, vorzugsweise zur Reduzierung der kalorischen Aufnahme und dabei bevorzugt zur therapeutischen Reduktion des Gewichtes,
   und/oder
(ii) zur Vermittlung eines Gefühls von Sättigung, vorzugsweise zur Reduzierung der kalorischen Aufnahme und dabei bevorzugt zur therapeutischen Reduktion des Gewichtes, und/oder
(iii) als Stimmungsaufheller,
wobei
n = 1, 2 bedeutet, wobei die resultierende(n) Doppelbindung(en) unabhängig voneinander als E-oder Z-Konfiguration vorliegen,
X = - OR¹ oder - NR²R³, mit
R¹ = Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
   und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
   oder
R² und R³ zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=.

Die vorliegende Erfindung betrifft ebenfalls die kosmetische Verwendung der genannten aromatischen Alkensäurederivate der Formel I zur Reduzierung des Appetits, vorzugsweise zur Reduzierung der kalorischen Aufnahme und/oder zur Vermittlung eines Gefühls von Sättigung, vorzugsweise zur Reduzierung der kalorischen Aufnahme und dabei bevorzugt zur kosmetischen Reduktion des Körpergewichtes.

In einer bevorzugten Ausführungsform werden die aromatischen Alkensäurederivate der Formel I zur Verwendung bei der Behandlung von Adipositas eingesetzt.

Ein weiterer Aspekt der vorliegenden Erfindung ist zudem die nicht-therapeutische Verwendung von aromatischen Alkensäurederivate der Formel I 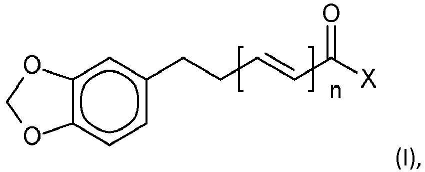
(i) zur Reduzierung des Appetits, vorzugsweise zur nicht-therapeutischen Reduzierung der kalorischen Aufnahme und dabei bevorzugt zur nicht-therapeutischen Reduktion des Gewichtes, und/oder
(ii) zur nicht-therapeutischen Vermittlung eines Gefühls von Sättigung, vorzugsweise zur nicht-therapeutischen Reduzierung der kalorischen Aufnahme und dabei bevorzugt zur nicht-therapeutischen Reduktion des Gewichtes, und/oder
(iii) als Stimmungsaufheller,
wobei
n = 1, 2 bedeutet, wobei die resultierende(n) Doppelbindung(en) unabhängig voneinander als E-oder Z-Konfiguration vorliegen,
X = - OR¹ oder - NR²R³, mit
R1 = Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl , Allyl, Prenyl oder Isoprenyl,
   und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
   oder
R² und R³ zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-, -CH=CH-CH=CH-CH=.

Bevorzugt werden die folgenden aromatische Alkensäurederivate der Formel I, 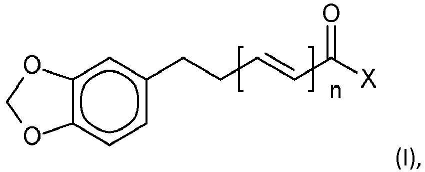 wobei
n= 1, 2, wobei die resultierende(n) Doppelbindung(en) zu mehr als 50% in E-Konfiguration vorliegt bzw. vorliegen,
X = - OR¹ oder -NR²R³, mit
R¹= Methyl, Ethyl
R² = Wasserstoff,
R³ = 2-Methylpropyl, 2-Methyl-1-butyl,
   oder
R² und R³ bilden zusammen einen gesättigten carbocyclischen Ring aus -CH₂-CH₂-CH₂-CH₂-CH₂-.
zur Anwendung in einem therapeutischen Verfahren oder in einem nicht-therapeutischen Verwendung, eingesetzt.

Besonders bevorzugt sind hierbei die aromatischen Alkensäurederivate der Formel I ausgewählt aus der Gruppe bestehend aus:
5-((1,3-Benzodioxol-5-yl)-N-isobutyl-pent-2-enamid (Dihydropiperlinguminin, Verbindung 1) 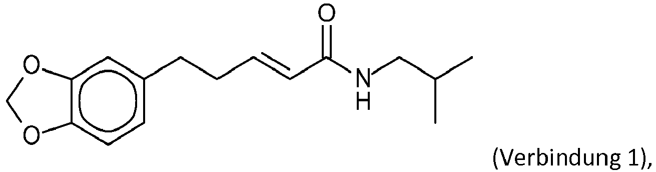
7-((1,3-Benzodioxol-5-yl)-N-isobutyl-hepta-2,4-dienamid (Chingchengenamide A, Verbindung 2): 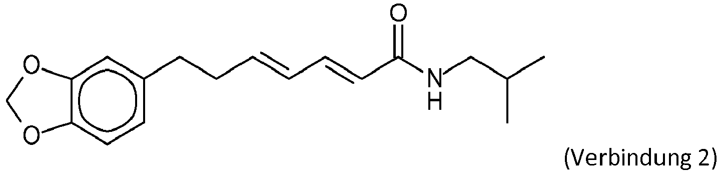
7-((1,3-Benzodioxol-5-yl)-1-(1-piperidyl)hepta-2,4-dien-1-on (Piperdardin, Verbindung 3) 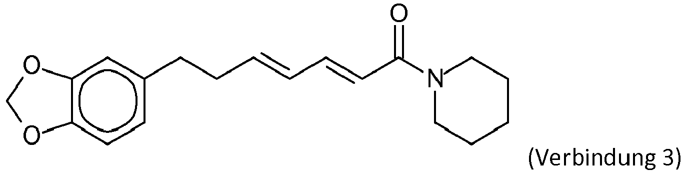
Methyl-7-(1,3-benzodioxol-5-yl)hepta-2,4-dienoat (Verbindung 4)

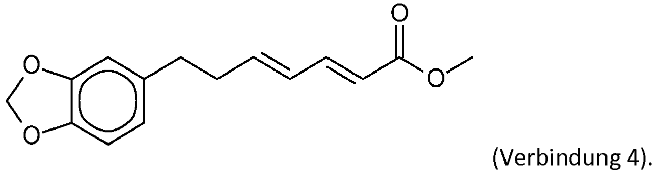

Die erfindungsgemäßen aromatischen Alkensäurederivate liegen vorzugsweise zu mehr als 50 %, besonders bevorzugt zu mehr als 80 %, insbesondere bevorzugt zu mehr als 90 % in der E-Konfiguration vor, wobei die aromatischen Alkensäurederivate vorzugsweise ausgewählt sind aus den Verbindungen 1 bis 4.

Bevorzugt werden die Verbindungen 1 bis 4 in kosmetischen Produkten zur Reduzierung des Appetits, vorzugsweise zur Reduzierung der kalorischen Aufnahme und/oder zur Vermittlung eines Gefühls von Sättigung, vorzugsweise zur Reduzierung der kalorischen Aufnahme und dabei bevorzugt zur kosmetischen Reduktion des Gewichtes, eingesetzt.

Ebenfalls bevorzugt werden die Verbindungen 1 bis 4 in Lebens-/Nahrungs-, Genuss- und / oder Nahrungsergänzungsmittel Produkten zur Reduzierung des Appetits, vorzugsweise zur Reduzierung der kalorischen Aufnahme und/oder zur Vermittlung eines Gefühls von Sättigung, vorzugsweise zur Reduzierung der kalorischen Aufnahme, vorzugsweise zur Reduktion des Körpergewichtes, eingesetzt.

Unter "kosmetisch" wird in der vorliegenden Erfindung die Anwendung von kosmetischen Mitteln verstanden, also Stoffe oder Gemische, die bestimmt sind für die Körper- und Schönheitspflege, bzw. für die Erhaltung, Wiederherstellung oder Verbesserung der Schönheit des menschlichen Körpers.

Die besonders bevorzugt genannten aromatischen Alkensäurederivate 1 bis 4 kommen alle natürlich, in bestimmten Pflanzenarten vor, wobei einige diese Pflanzen direkt oder in Form von Extrakten und Zubereitungen in Nahrungsmitteln eingesetzt werden können. Verbindung 1 wurde z.B. in *Piper longum* (Stangenpfeffer, Tabuneng, W., Bando, H., Amiya, T., 1983. Chemical & Pharmaceutical Bulletin 31, 3562-3565) gefunden, Verbindung 2 in *P. nigrum* (Friedman, M., Levin, C. E., Lee, S. U., Lee, J. S., Ohnisi-Kameyama, M., Kozukue, N., 2008. J Agric Food Chem 56, 3028-3036) und Verbindung 3 in *Piper nigrum* (Schwarzer Pfeffer, Kapoor, I. P., Singh, B., Singh, G., De Heluani, C. S., De Lampasona, M. P., Catalan, C. A., 2009. J Agric Food Chem 57, 5358-5364) beschrieben. Verbindung 4 wurde bisher nicht in der Literatur als Naturstoff beschrieben.

Demgemäß ist ein Pflanzenextrakt, welche Verbindung 4 (Methyl-7-(1,3-benzodioxol-5-yl)hepta-2,4-dienoat) umfasst und Verbindung 4 (Methyl-7-(1,3-benzodioxol-5-yl)hepta-2,4-dienoat) selbst, ebenfalls Gegenstand der vorliegen Erfindung.

Die Pflanzenextrakte enthalten eine oder mehrere der bevorzugt genannten aromatischen Alkensäurederivate und sind ebenfalls Sinne der Erfindung einsetzbar, beispielsweise Pfefferextrakt *(Piper ssp.,* insbesondere *P. nigrum, P. falconeri, P. boehmeriaefolium, P. tuberculatum, P. sintenensis, P. longum, P. chaba* oder *P. guineense),* Extrakte aus Fagara-Arten (*Fagara macrophylla)* sowie Extrakte aus *Macropiper excelsum.*

Die pflanzlichen Extrakte enthaltend eines oder mehrere der aromatischen Alkensäurederivate der Formel I, insbesondere der Verbindungen 1 bis 4. Die Alkensäurederivate der Formel I können aus den entsprechenden frischen oder getrockneten Pflanzen oder Pflanzenteilen, insbesondere aber aus weißen, grünen oder schwarzen Pfefferkörnern (P. *nigrum),* Stangenpfeffer *(P. longum),* Pflanzenteilen der Fagara-Arten oder den Blättern, Samen oder Fruchtständen von *Macropiper excelsum* gewonnen werden. Üblicherweise werden die getrockneten Pflanzenteile (z.B. frische oder getrocknete Wurzeln, Früchte, Samen, Rinde, Holz, Stängel, Blätter oder Blüten[teile]), vorzugsweise in zerkleinerter Form, mit einem für Nahrungs- und Genussmittel geeigneten Lösungsmittel bei Temperaturen von 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise enzymatisch behandelt werden, mit Säure (z.B. unter Druck), mit sauren Ionentauschern oder mit Wasserdampf behandelt werden, meist bei Drücken von 0,01 mbar bis 100 bar, vorzugsweise bei 1 mbar bis 20 bar, und/oder in einem für Nahrungs- und Genussmittel geeigneten Lösungs-mittel aufgenommen werden. Bevorzugt sind Extrakte, in denen die aromatischen Alkensäurederivate den Hauptanteil bezogen auf die Gesamtmenge der Amid-artigen Verbindungen darstellen, beispielsweise 1 bis 90 %, bevorzugt 5 Gew.-%, besonders bevor-zugt 10 Gew.-%, besonders bevorzugt 50 Gew.-%, ganz besonders bevorzugt 80 Gew.-% bezogen auf die Gesamtmenge der Amid-artigen Verbindungen darstellt.

Insbesondere für Nahrungs- und Genussmittel zur Extraktion geeignete Lösungsmittel sind Wasser, Ethanol, Methanol, 1,2-Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Bevorzugte Hilfs- oder Trägerstoffe sind Maltodextrin, Stärke, natürliche oder künstliche Polysaccharide und/oder Pflanzengummen wie modifizierte Stärken oder Gummi Arabi-cum, für die Aromakompositionen zugelassene Lösungsmittel wie z.B. Ethanol, 1,2-Propylenglycol, Wasser, Glycerin, Triacetin, Pflanzenöltriglyceride, färbende Mittel, z.B. zugelassene Lebensmittelfarbstoffe, färbende Pflanzenextrakte, Stabilisatoren, Konservierungsstoffe, Antioxidantien, Viskositäts-beeinflussende Stoffe.

### Die vorliegende Erfindung betrifft auch ein Verfahren zur nicht-therapeutischen

(i) zur Reduzierung des Appetits, vorzugsweise zur nicht-therapeutischen Reduzierung der kalorischen Aufnahme und dabei bevorzugt zur nicht-therapeutischen Reduktion des Gewichtes, und/oder
(ii) zur nicht-therapeutischen Vermittlung eines Gefühls von Sättigung, vorzugsweise zur nicht-therapeutischen Reduzierung der kalorischen Aufnahme und dabei bevorzugt zur nicht-therapeutischen Reduktion des Gewichtes, und/oder
(iii) zur Aufhellung der Stimmung,
   mit folgendem Schritt:
   - Verabreichen einer (a) den Appetit reduzierenden und/oder (b) ein Gefühl von Sättigung bewirkenden und/oder (c) einer stimmungsaufhellenden Menge von einem oder mehreren aromatischen Alkensäurederivaten der Formel I, 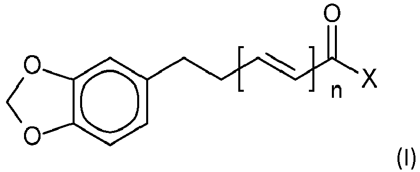 wobei
      n = 1, 2 bedeutet, wobei die resultierende(n) Doppelbindung(en) unabhängig voneinander als E-oder Z-Konfiguration vorliegen,
      X = - OR¹ oder - NR²R³, mit
      R¹= Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl,2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
         und wobei
      R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
         oder
      R² und R³ zusammen einen gesättigten oder ungesättigten carbocyclischen Ring ausbilden ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2- oder -CH=CH-CH=CH-CH=.

Hierbei werden bevorzugt die aromatischen Alkensäurederivat-Verbindungen 1 bis 4 eingesetzt, wobei 1, 2, 3 oder alle 4 Verbindungen alleine oder im Gemisch vorliegen können.

Die aromatischen Alkensäurederivate der Formel I werden bevorzugt in Mitteln oder Zubereitungen eingearbeitet, welche bevorzugt oral konsumierbare Produkte sind. Ein solches Mittel oder eine solche Zubereitung umfasst vorzugsweise mindestens ein, zwei, drei oder vier aromatische Alkensäurederivate der Formel I, vorzugsweise Verbindungen 1 bis 4.

Demgemäß ist ein weiterer Gegenstand der vorliegenden Erfindung eine Zubereitung, umfassend mindestens ein aromatisches Alkensäurederivat der Formel I 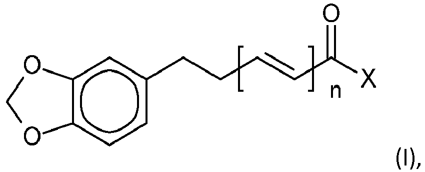 zur Anwendung in einem therapeutischen Verfahren oder in einer nicht-therapeutischen Verwendung,
(i) zur Reduzierung des Appetits, vorzugsweise zur nicht-therapeutischen Reduzierung der kalorischen Aufnahme und dabei bevorzugt zur nicht-therapeutischen Reduktion des Gewichtes, und/oder
(ii) zur nicht-therapeutischen Vermittlung eines Gefühls von Sättigung, vorzugsweise zur nicht-therapeutischen Reduzierung der kalorischen Aufnahme und dabei bevorzugt zur nicht-therapeutischen Reduktion des Gewichtes, und/oder
   und/oder
(iii) als Stimmungsaufheller,
   wobei
   n = 1, 2 bedeutet, wobei die resultierende(n) Doppelbindung(en) unabhängig voneinander als E-oder Z-Konfiguration vorliegen,
   X=-OR¹ oder - NR²R³, mit
   R¹= Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
      und wobei
   R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
      oder
   R² und R³ zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=.

Bevorzugt werden in einer solchen Zubereitung zur Anwendung in einem therapeutischen Verfahren oder in einer nicht-therapeutischen Verwendung, aromatische Alkensäurederivate eingesetzt,
wobei
n= 1, 2, wobei die resultierende(n) Doppelbindung(en) zu mehr als 50% in E-Konfiguration vorliegt bzw. vorliegen,
X = - OR1 oder -NR2R3, mit
R¹ = Methyl, Ethyl
R²= Wasserstoff,
R³ = 2-Methylpropyl, 2-Methyl-1-butyl,
   oder
R2 und R3 bilden zusammen einen gesättigten carbocyclischen Ring bestehend aus -CH2-CH2-CH2-CH2-CH2-, aus
   und
wobei die aromatischen Alkensäurederivate zu mehr als 50 % in der E-Konfiguration vorliegen.

Bevorzugt werden in einer solchen Zubereitung zur Anwendung in einem therapeutischen Verfahren oder in einer nicht-therapeutischen Verwendung, aromatische Alkensäurederivate eingesetzt, die ausgewählt sind aus der Gruppe bestehend aus:
5-((1,3-Benzodioxol-5-yl)-N-isobutyl-pent-2-enamid (Verbindung 1),
7-((1,3-Benzodioxol-5-yl)-N-isobutyl-hepta-2,4-dienamid (Verbindung 2),
7-((1,3-Benzodioxol-5-yl)-1-(1-piperidyl)hepta-2,4-dien-1-on (Verbindung 3),
Methyl-7-(1,3-benzodioxol-5-yl)hepta-2,4-dienoat (Verbindung 4).

Hierbei liegen die Verbindungen 1 bis 4 bevorzugt zu mehr als 50 %, besonders bevorzugt zu mehr als 80 %, insbesondere bevorzugt zu mehr als 90 % in der E-Konfiguration vor.

Die besonders bevorzugt aromatischen Alkensäurederivate 1 bis 4 können als Pflanzenextrakte beispielsweise als Pfefferextrakt (*Piper ssp.,* insbesondere *P. nigrum, P. falconeri , P. boehmeriaefolium, P. tuberculatum, P. sintenensis, P. longum, P. chaba* oder *P. guineense*), Extrakte aus Fagara-Arten (Fagara macrophylla) sowie Extrakte aus *Macropiper excelsum* gewonnen werden. Neben den genannten aromatischen Alkensäurederivate sind auch bestimmte nicht-aromatische Alkamide in den Pflanzenextrakten zu finden, insbesondere das 2E,4E-Decadiensäure-N-isoubutylamid (*trans*-Pellitorin). Das trans-Pellitorin ist ebenfalls bekannt als Stoff, welche Sättigungs-reduzierende Effekte zeigt. Daher ist das parallele Vorkommen in den Extrakten von z.B. *Piper nigrum, Piper longum* oder *Macropiper excelsum* von großem Vorteil, so dass eine Kombination von trans-Pellitorin mit aromatischen Alkensäurederivate eine bevorzugte Ausführungsform darstellt.

Ebenso ist eine Kombination mit Capsaicinoiden, insbesondere mit dem Nonivamid (N-Nonanoylvanillylamin), welche ebenfalls zur Reduzierung des Appetits und/oder zur Vermittlung eines Gefühls von Sättigung und/oder zur Aufhellung der Stimmung eingesetzt werden kann, möglich.

Daher besteht eine bevorzugte Ausführungsform der vorliegenden Anmeldung in Zubereitungen zur Anwendung in einem therapeutischen Verfahren oder in einer nicht-therapeutischen Verwendung, wobei die Zubereitung, mindestens ein aromatisches Alkensäurederivat nach Formel I und/ oder 2E,4E-Decadiensäure-N-isoubutylamid (trans-Pellitorin) und/oder Nonivamid (N-Nonanoylvanillylamin) und/oder Capsaicin umfasst.

Bei allen Ausführungsformen sind Einzelstoffe oder Mischungen der aromatischen Alkensäurederivate der Formel I bevorzugt, die nur einen unterschwelligen Aromawert in der Einsatzkonzentration zeigen, d.h. bevorzugt werden die Verbindungen um den Erkennnungsschwellenwert herum oder niedriger eingesetzt (bezogen auf das verzehrsfertige Nahrungsmittel). Das gilt ebenfalls auch für die Verbindungen Nonivamid (N-Nonanoylvanillylamin) und trans-Pellitorin sowie Capsaicinoiden, wenn diese in einer solchen Zubereitung vorliegen.

Demgemäß ist eine bevorzugte Ausführungsform eine Zubereitung zur Anwendung in einem therapeutischen Verfahren oder in einer nicht-therapeutischen Verwendung, wobei die Menge an aromatische(n) Alkensäurederivat(en) 2E,4E-Decadiensäure-N-isoubutyl-amid (trans-Pellitorin) und/oder Nonivamid (N-Nonanoylvanillylamin) und/oder Capsaicin umfasst.

Überraschenderweise hat sich gezeigt, dass die erfindungsgemäßen aromatischen Alkensäurederivate der Formel I in der Lage sind, bereits in einem Konzentrationsbereich von 0,01 µM bis 10 µM (ca. 0,02 mg/kg bis ca. 2 mg/kg) die Serotonin-Freisetzung in Neuronen um maximal bis zu ca. 300 % gegen die jeweilige Kontrolle anzuregen.

Die jeweiligen Konzentrationen der aromatischen Alkensäurederivate sind im Vergleich zu typischen Einsatzkonzentrationen, um einen Geschmackseffekt zu erzielen, gering, es ist also nicht mit stark wahrnehmbaren Aromaeffekten zu rechnen.

Demgemäß ist eine bevorzugte Ausführungsform eine Zubereitung zur Anwendung in einem therapeutischen Verfahren oder in einer nicht-therapeutischen Verwendung, wobei die Gesamtkonzentration der aromatischen Alkensäurederivate der Formel I in der Zubereitung in einem Bereich von weniger als 20 mg/kg, bevorzugt in einer Konzentration von 5 mg/kg oder weniger, besonders bevorzugt in einer Konzentration von 2 mg/kg oder weniger, vorliegt, bezogen auf die Gesamtmasse der Zubereitung.

Nonivamid (N-Nonanoylvanillylamin) und trans-Pellitorin und die erfindungsgemäßen aromatischen Alkensäurederivaten der Formel I weisen gleichwertige Aktivitäten zur Reduzierung des Appetits, vorzugsweise zur nicht-therapeutischen Reduzierung der kalorischen Aufnahme und dabei bevorzugt zur nicht-therapeutischen Reduktion des Gewichtes, und/oder zur nicht-therapeutischen Vermittlung eines Gefühls von Sättigung, vorzugsweise zur nicht-therapeutischen Reduzierung der kalorischen Aufnahme und dabei bevorzugt zur nicht-therapeutischen Reduktion des Gewichtes, und/oder zur Aufhellung der Stimmung, auf.

Die Kombination aus 2E,4E-Decadiensäure-N-isoubutylamid (trans-Pellitorin) und/ oder Nonivamid (N-Nonanoylvanillylamin) und/oder Capsaicin und den erfindungsgemäßen aromatischen Alkensäurederivaten der Formel I, insbesondere den Verbindungen 1 bis 4, einzeln oder im Gemisch, ist daher besonders vorteilhaft, da insbesondere die erfindungsgemäßen aromatischen Alkensäurederivate bei der oben genannten Dosierung einen, gegenüber Nonivamid oder trans-Pellitorin oder Capsaicinoiden, sensorisch niedrigeren Wert, bei gleichbleibender erfindungsgemäßer Wirkung, aufweisen. Dies ist insbesondere vorteilhaft, da die aromatischen Alkensäurederivate, insbesondere Verbindungen 1 bis 4, in Zubereitungen, wie oral konsumierbaren Produkten, in einer höheren Menge einsetzbar sind oder einen Teil des Nonivamids oder einen Teil des trans-Pellitorin bzw. der Capsaicinoiden ersetzen können, ohne den Geschmack negativ zu beeinflussen bzw. sogar den durch Nonivamid oder trans-Pellitorin oder Capsaicinoiden ausgelösten Geschmackswert unter deren Schwellenwert senken können.

Bevorzugt ist eine solche Zubereitung ein oral konsumierbares Produkt oder Bestandteil eines oral konsumierbaren Produktes. Insbesondere bevorzugt ist eine solche Zubereitung ausgewählt aus der Gruppe bestehend aus Kosmetika, flüssige oder feste Genussmitteln, Lebensmitteln, Halbfertigwaren, Futtermitteln und Arzneimitteln.

Vorzugsweise werden die genannten erfindungsgemäßen aromatischen Alkensäurederivate zur erfindungsgemäßen Anwendung in einem therapeutischen Verfahren bzw. in einer erfindungsgemäßen nicht-therapeutischen, vorzugsweise kosmetischen Verwendung eingesetzt, wobei die erfindungsgemäßen aromatischen Alkensäurederivate vorzugsweise alleine oder als Gemisch Bestandteil eines oral konsumierbaren Produktes (insbesondere eines kosmetischen Produktes, Lebensmittels, Futtermittels oder Arzneimittels) eingesetzt wird, wobei die erfindungsgemäßen aromatischen Alkensäurederivate der Formel I in einer Konzentration von 20 mg/kg oder weniger vorliegt, bezogen auf die Gesamtmasse des oral konsumierbaren Produktes.

Bevorzugt ist ein erfindungsgemäß oral konsumierbares Produkt ausgewählt aus der Gruppe bestehend aus kosmetischen Produkten, Genussmitteln, Lebensmitteln (insbesondere flüssige oder feste, einschließlich Halbfertigwaren), Futtermitteln und Arzneimitteln (pharmazeutische Zubereitungen).

Demgemäß ist ein Gegenstand der Erfindung eine pharmazeutische Zusammensetzung, umfassend mindestens ein aromatisches Alkensäurederivate der Formel I als Wirkstoff, vorzugsweise mindestens eine oder mehrere Verbindungen 1 bis 4.

Demgemäß ist ein weiterer Gegenstand der Erfindung eine kosmetische Zusammensetzung, umfassend mindestens ein aromatisches Alkensäurederivate der Formel I, vorzugsweise mindestens eine oder mehrere Verbindungen 1 bis 4.

Demgemäß ist ein weiterer Gegenstand der Erfindung ein Genuss- oder Lebensmittel oder Nahrungsergänzungsmittel, umfassend mindestens ein aromatisches Alkensäurederivate der Formel I, vorzugsweise mindestens eine oder mehrere Verbindungen 1 bis 4.

Im Rahmen des vorliegenden Textes umfasst der Begriff "Lebensmittel" eine Vielzahl von Produkten. Unter den Begriff Lebensmittel fallen insbesondere Produkte, wie sie weiter unten im Zusammenhang mit erfindungsgemäßen Lebensmitteln diskutiert werden.

Der Begriff "Lebensmittel" umfasst insbesondere Produkte, die gemäß der VERORDNUNG (EG) Nr. 178/2002 DES EUROPÄISCHEN PARLAMENTS UND DES RATES vom 28. Januar 2002 Lebensmittel sind. Gemäß dieser Verordnung sind "Lebensmittel" alle Stoffe oder Erzeugnisse, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden. Zu "Lebensmitteln" zählen auch Getränke, Kaugummi sowie alle Stoffe - einschließlich Wasser -, die dem Lebensmittel bei seiner Herstellung oder Ver- oder Bearbeitung absichtlich zugesetzt werden.

Der Begriff "Futtermittel" umfasst im Rahmen des vorliegenden Textes alle Formen von Tiernahrung. Eine Vielzahl der nachstehend genannten Lebensmittel kann auch als Futtermittel eingesetzt werden.

Der Begriff Arzneimittel ist gleichzusetzen mit den Begriff pharmazeutische Zusammensetzung bzw. Produkt. Es versteht sich dass der Begriff "Arzneimittel/ pharmazeutische Zusammensetzung/ pharmazeutisches Produkt" im Rahmen des vorliegenden Textes Stoffe oder Stoffzusammensetzungen umfasst, die als Mittel mit Eigenschaften zur Heilung oder zur Verhütung menschlicher oder tierischer Krankheiten bestimmt sind oder aber im oder am menschlichen oder tierischen Körper verwendet oder einem Menschen bzw. Tier verabreicht werden können, um entweder die menschlichen bzw. tierischen physiologischen Funktionen durch eine pharmakologische, immunologische oder metabolische Wirkung wiederherzustellen, zu korrigieren oder zu beeinflussen oder eine medizinische Diagnose zu erstellen. Pharmazeutische Zusammensetzungen bzw. Produkte können auch im Einzelfall zu nicht-therapeutischen, insbesondere kosmetischen Zwecken eingesetzt werden.

Es versteht sich, dass Lebensmittel oder Futtermittel durch den Zusatz von Stoffen oder Stoffzusammensetzungen, die als Mittel mit Eigenschaften zur Heilung oder zur Verhütung menschlicher oder tierischer Krankheiten bestimmt sind, in entsprechende Arzneimittel überführt werden können.

Vorzugsweise weisen die erfindungsgemäßen Zubereitungen mindestens 1, 2, 3 oder 4 aromatische Alkensäurederivate der Formel I auf, welche bevorzugt ausgewählt sind aus der Gruppe der Verbindung 1, Verbindung2, Verbindung 3 oder Verbindung 4. Ebenfalls ist es vorteilhaft, wenn alle 4 Verbindungen in einer solchen Zubereitung vorliegen. Bei der Bestimmung der Gesamtkonzentration der aromatischen Alkensäurderivate in einer Zubereitung ist die Summe der Konzentrationen der einzelnen Verbindungen gemeint.

Die vorliegende Erfindung umfasst demgemäß auch oral konsumierbare Produkte, umfassend mindestens ein, zwei, drei oder vier aromatische Alkensäurederivate der Formel I. Vorzugsweise umfasst ein solches oral konsumierbares Produkt mindestens 1, mindestens 2, mindestens 3 oder alle vier Verbindungen 1 bis 4. Vorzugsweise liegen die aromatischen Alkensäurederivate zu mehr als 50 %, besonders bevorzugt zu mehr als 80 %, insbesondere bevorzugt zu mehr als 90 % in der E-Konfiguration vor.

Eine nicht-therapeutische Verwendung oder ein nicht-therapeutisches Verfahren im Sinne der vorliegenden Erfindung betrifft Verfahren, die nicht zur chirurgischen oder therapeutischen Behandlung des menschlichen oder tierischen Körpers gedacht sind. Vorzugsweise bezieht sich der Begriff "nicht-therapeutisch" in der vorliegenden Erfindung auf kosmetische Verfahren/kosmetische Anwendungen/ kosmetische Produkte sowie Lebens-, Genuss-, Futtermittel und Nahrungsergänzungsmittel, in der die erfindungsgemäßen aromatischen Alkensäurederivate, insbesondere Verbindungen 1 bis 4, einformuliert werden können, um den Appetit zu reduzieren oder ein Sättigungsgefühl zu vermitteln oder als Stimmungsaufheller, vorzugsweise um einen kosmetischen Nutzen hinsichtlich der Körpergewichtsreduzierung zu erreichen.

In einer besonders bevorzugten erfindungsgemäßen Zubereitung zur Anwendung sowie in erfindungsgemäßen nicht-therapeutischen Verwendungen einer Zubereitung oder in erfindungsgemäßen kosmetischen Verwendungen einer Zubereitung, umfasst die Zubereitung 2E,4E-Decadiensäure-N-isoubutylamid (trans-Pellitorin) und/oder Nonivamid (N-Nonanoylvanillylamin) und/oder Capsaicin

Ein weiterer Aspekt der vorliegenden Erfindung betrifft die Verwendung einer Zubereitung umfassend eines oder mehrerer der benannten aromatischen Alkensäurederivate und einen oder mehrere weitere Stoffe in einem therapeutischen Verfahren
(i) zur Reduzierung des Appetits, vorzugsweise zur Reduzierung der kalorischen Aufnahme und dabei bevorzugt zur therapeutischen Reduktion des Gewichtes,
   und/oder
(ii) zur Vermittlung eines Gefühls von Sättigung, vorzugsweise zur Reduzierung der kalorischen Aufnahme und dabei bevorzugt zur therapeutischen Reduktion des Gewichtes, und/oder
(iii) als Stimmungsaufheller,
wobei in dem therapeutischen Verfahren das in der Zubereitung enthaltene aromatische Alkensäurederivat oder die in der Zubereitung enthaltenen aromatischen Alkensäurederivate so eingesetzt werden, dass es/sie (a) den Appetit reduzieren und/oder (b) ein Gefühl von Sättigung hervorrufen und/oder(c) die Stimmung aufhellt.

In einer besonders bevorzugten erfindungsgemäßen Zubereitung zur Anwendung und in besonders bevorzugten erfindungsgemäßen nicht-therapeutischen Verwendungen einer Zubereitung sowie in besonders bevorzugten erfindungsgemäßen kosmetischen Verwendungen einer Zubereitung, handelt es sich bei den weiteren Stoffen um vorzugsweise sprühgetrocknete Stoffe, die zum Verzehr geeignete Bestandteile, feste Trägerstoffe und ggf. bestimmte Aromastoffe bzw. Aromakompositionen umfassen.

In weiteren besonders bevorzugten erfindungsgemäßen Zubereitungen zur Anwendung und in besonders bevorzugten erfindungsgemäßen Verwendungen einer Zubereitung, handelt es sich bei den weiteren Stoffen um zum Verzehr geeignete feste Trägerstoffe.

Vorteilhafte Trägerstoffe in diesen bevorzugten erfindungsgemäßen (vorzugsweise sprühgetrockneten) Zubereitungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Maisbasierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zubereitungen, die neben dem erfindungsgemäß zu verwendenden aromatische Alkensäurederivat oder mehreren aromatischen Alkensäurederivate noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Zubereitungen, die mittels Sprühtrocknung hergestellt wurden, besitzen eine mittlere Partikelgröße im Bereich von 30 bis 300 µm und eine Restfeuchte von kleiner oder gleich 5 Gew.-%.

Eine erfindungsgemäße Zubereitung ist vorzugsweise eine (bevorzugt sprühgetrocknete) Zubereitung, umfassend
(i) mindestens ein aromatisches Alkensäurederivat der Formel I in einer wirksamen Menge,
(ii) mindestens einen festen Trägerstoff,
(iii) mindestens ein Aromastoff.
Dabei gilt hinsichtlich der Verbindungen und Mischungen das oben Gesagte entsprechend.

Der oder zumindest einer der Aromastoffe der Komponente (iii) ist dabei vorzugsweise eine sensorisch wirksame Komponente und wird bevorzugt eingesetzt in einer Konzentration, die größer ist als sein Reizschwellenwert.

Beispiele für Aromastoffe der Komponente (iii), die Bestandteil der Zubereitung sein können, finden sich z.B. in H. Surburg, J. Panten, Common Fragrance and Flavor Materi-als, 5th. Ed., Wiley-VCH, Weinheim 2006.

Aromastoffe der Komponente (iii) können in Form von Aromakompositionen eingesetzt werden, die wiederum in Form von Reaktionsaromen (Maillard-Produkte) und/oder Ex-trakten bzw. ätherischen Ölen von Pflanzen oder Pflanzenteilen bzw. Fraktionen davon eingesetzt werden können.

In einem weiteren Aspekt betrifft die vorliegende Erfindung oral konsumierbare Produkte, umfassend mindestens ein aromatisches Alkensäurederivat der Formel I 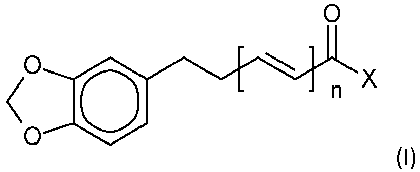 wobei
n = 1, 2 bedeutet, wobei die resultierende(n) Doppelbindung(en) unabhängig voneinander als E-oder Z-Konfiguration vorliegen,
X=-OR¹ oder - NR²R³, mit
R¹= Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
   und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
   oder
R² und R³ zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2-,-CH=CH-CH=CH-CH=,
wobei die Menge des/der verwendeten aromatischen Alkensäurederivate(s) in einer den Appetit reduzierenden und/ oder ein Gefühl von Sättigung bewirkenden Konzentration eingesetzt wird.

Weiterhin betrifft die Erfindung ein oral konsumierbares Produkt (insbesondere Lebensmittel), umfassend mindestens ein, bevorzugt zwei oder drei oder vier aromatische Alkensäurederivate der Formel I und einen oder mehrere weitere Stoffe, wobei das aromatische Alkensäurederivat oder eine Mischung mehrerer aromatischen Alkensäurederivate
- in einer den Appetit reduzierenden und/oder ein Gefühl von Sättigung bewirkenden Konzentration
   und/oder
   in einer die Stimmung aufhellenden Konzentration und gleichzeitig
- in einer Konzentration von 20 mg/kg oder weniger, bezogen auf die Gesamtmasse des oral konsumierbaren Produktes, vorzugsweise in einer Konzentration von 5 mg/kg oder weniger, besonders bevorzugt in einer Konzentration von 2 mg/kg oder weniger vorliegt und gleichzeitig
- in einer Konzentration von zumindest 0,0001 mg/kg oder mehr, bezogen auf die Gesamtmasse des oral konsumierbaren Produktes, vorzugsweise in einer Konzentration von 0,001 mg/kg oder mehr, ganz besonders bevorzugt in einer Konzentration von 0,005 mg/kg oder mehr vorliegt.

Besonders bevorzugt ist ein erfindungsgemäßes oral konsumierbares Produkt, bei dem es sich um ein Lebensmittel, Futtermittel und/oder Arzneimittel handelt.

Um eine Reduktion des Körpergewichtes beim Konsumenten zu unterstützen, hat es sich als sinnvoll erwiesen, die potentielle kalorische Aufnahme zu begrenzen. Dies kann zum einen dadurch erfolgen, dass die erfindungsgemäße Verwendung des aromatischen Alkensäurederivats oder einer Mischung der aromatischen Alkensäurederivate in oral konsumierbaren Produkten (insbesondere Lebensmitteln, Futtermitteln und Arzneimitteln) ein Gefühl von Sättigung vermittelt und gleichzeitig den Appetit reduziert, sowie zusätzlich die Absorption von energieliefernden Nährstoffen aus dem Darm verringert. Dadurch wird der Konsument nicht nur dazu veranlasst, den Verzehr von energiehaltigen Produkten zu begrenzen, sondern ihm steht von der oral aufgenommenen Energiemenge durch eine verringerte Absorption auch weniger Nahrungsenergie zur Verfügung. Neben der durch die aromatischen Alkensäurederivate verursachten geringeren Aufnahme und Akkumulation von Lipiden lässt die sich kalorische Aufnahme zusätzlich reduzieren, wenn erfindungsgemäße oral konsumierbare Produkte (insbesondere Lebensmittel, Futtermittel bzw. Arzneimittel) zum Verzehr angeboten werden, die ihrerseits bereits eine geringe Energiedichte aufweisen.

Ein bevorzugtes erfindungsgemäßes oral konsumierbares Produkt (insbesondere Lebensmittel, Futtermittel und Arzneimittel) enthält nicht mehr als 200 kcal/100g des oral konsumierbaren Produktes, bevorzugt nicht mehr als 100 kcal/100g, besonders bevorzugt nicht mehr als 40 kcal/100g.

Bevorzugte erfindungsgemäße oral konsumierbare Produkte (insbesondere Lebensmittel, Futtermittel oder Arzneimittel) sind jegliche zum Verzehr geeignete der Ernährung, der Mundpflege oder dem Genuss dienende Zubereitungen oder Zusammensetzungen und sind regelmäßig Produkte, die dazu bestimmt sind, in die menschliche bzw. tierische Mundhöhle eingebracht zu werden, dort eine bestimmte Zeit zu verbleiben und anschließend entweder verzehrt (z.B. verzehrfertige Lebensmittel oder Futtermittel, siehe auch weiter unten) oder wieder aus der Mundhöhle entfernt zu werden (z.B. Kaugummis oder Mundpflegeprodukte oder medizinische Mundspülungen). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen oder Tier aufgenommen zu werden. Hierzu zählen auch Stoffe, die oral konsumierbaren Produkten (insbesondere Lebensmittel, Futtermittel und Arzneimittel) bei ihrer Herstellung, Verarbeitung oder Bearbeitung zugesetzt werden und dazu vorgesehen sind, in die menschliche oder tierische Mundhöhle eingebracht zu werden.

Zu den erfindungsgemäßen oral konsumierbaren Produkten (insbesondere Lebensmittel, Futtermittel und Arzneimittel) zählen auch Stoffe, die dazu bestimmt sind, in unverändertem, zubereitetem oder verarbeitetem Zustand vom Menschen oder Tier geschluckt und dann verdaut zu werden; zu den erfindungsgemäßen oral konsumierbaren Produkten gehören insoweit auch Umhüllungen, Überzüge oder sonstige Umschließungen, die dazu bestimmt sind, mit verschluckt zu werden, oder bei denen ein Verschlucken vorauszusehen ist. Der Begriff "oral konsumierbares Produkt" umfasst verzehrfertige Lebensmittel und Futtermittel, d.h. Lebensmittel oder Futtermittel, die hinsichtlich der für den Geschmack maßgeblichen Substanzen bereits vollständig zusammengesetzt sind. Unter den Begriffen "verzehrfertiges Lebensmittel" oder "verzehrfertiges Futtermittel" fallen auch Getränke sowie feste oder halbfeste verzehrfertige Lebensmittel oder Futtermittel. Als Beispiele seien Tiefkühlprodukte genannt, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln oder Futtermitteln.

Bevorzugte erfindungsgemäße oral konsumierbare Produkte (insbesondere Lebensmittel und Futtermittel) umfassen auch "Halbfertigwaren". Unter einer Halbfertigware ist im Zusammenhang des vorliegenden Textes ein oral konsumierbares Produkt zu verstehen, welches aufgrund eines sehr hohen Gehaltes an Aroma- und Geschmacksstoffen für die Verwendung als verzehrfertiges oral konsumierbares Produkt (insbesondere Lebensmittel oder Futtermittel) ungeeignet ist. Erst durch Mischen mit mindestens einem weiteren Bestandteil (d.h. durch Verringern der Konzentration der betreffenden Aroma- und Ge-schmacksstoffe) und gegebenenfalls weitere Prozessschritte (z.B. Erwärmen, Gefrieren) wird die Halbfertigware in ein verzehrfertiges oral konsumierbares Produkt (insbesondere Lebensmittel oder Futtermittel) überführt. Als Beispiel für Halbfertigwaren seien hier Tütensuppen, Backaromen und Puddingpulver genannt.

Zu erfindungsgemäßen oral konsumierbaren Produkten (insbesondere Lebensmitteln, Futtermitteln oder Arzneimitteln) zählen auch "Mundpflegeprodukte". Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Aero-sole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugt umfasst ein erfindungsgemäßes oral konsumierbares Produkt (insbesondere Lebensmittel oder Futtermittel), eine oder mehrere der Ernährung oder dem Genuss dienende Zubereitungen. Hierunter fallen insbesondere (kalorienreduzierte) Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Dragees, Hart- und Weichkaramellen, Kaugummi), nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegartes Gemüse, eingekochtes Gemüse), Knabberartikel (z.B. gebackene oder frittierte Kartoffel-chips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen und als Nahrungsergänzungsmittel vorliegen.

Besonders bevorzugt sind kalorienreduzierte Süßwaren (z.B. Müsliriegelprodukte, Fruchtgummi, Dragees, Hart- und Weichkaramellen, Kaugummi), nicht-alkoholische Getränke (z.B. Kakao, Grüntee, Schwarztee, mit (Grün-, Schwarz-)Tee-Extrakten ange-reicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäf-te, Frucht- oder Gemüsesaftzubereitungen), Instantgetränke (z.B. Instant-Kakao-Getränke, Instant-TeeGetränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Trockenmilchpulver, Molke, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen, -tabletten oder -sachets, sonstige Zube-reitungen zum Süßen oder Weißen von Getränken oder anderen Nahrungsmitteln.

Insbesondere bevorzugt sind kalorienreduzierte oder kalorienfreie Süßwaren (z.B. Müsli-riegelprodukte, Fruchtgummi, Dragees, Hartkaramellen, Kaugummi), nicht-alkoholische Getränke (z.B. Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zucker-arme oder -freie Limonaden, isotonische Getränke, Nektare, Obst- und Gemüsesäfte, Frucht- oder Gemüsesaftzubereitungen), Instantgetränke (z.B. Instant-(Grün-, Schwarz, Roibos, Kräuter)Tee-Getränke), Getreideprodukte (z.B. zuckerarme oder -freie Frühstückscerealien, Müsliriegel), Milchprodukte (z.B. fettreduzierte oder fettfreie Milchgetränke, Joghurt, Kefir, Molke, Buttermilch), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, oder daraus gefertigte Produkte und Mischungen mit Fruchtzubereitungen und fakultativ Aromen) oder Süßstoffzubereitungen, -tabletten oder -sachets.

Die Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die Halbfertigwaren dienen in der Regel zur Herstellung von der Ernährung oder dem Genuss dienenden gebrauchs- oder verzehrfertigen Zubereitungen.

Weitere Bestandteile einer der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Ge-müse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorri-genzien für unangenehme Geschmackseindrücke, weitere Geschmacksmodulatoren für weitere, in der Regel nicht unangenehme Geschmackseindrücke, andere geschmacks-modulierende Stoffe (z.B. Inositolphosphat, Nucleotide wie Guanosinmonophosphat, Adenosin-monophosphat oder andere Stoffe wie Natriumglutamat oder 2-Phen-oxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbin-säure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürli-che oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße oral konsumierbare Produkte (insbesondere Lebensmittel, Futtermittel und Arzneimittel), z.B. solche in Form von Zubereitungen oder Halbfertigwaren, eine Aromakomposition, um den Geschmack und/oder den Geruch abzurunden und zu verfeinern. Eine Zubereitung kann als Bestandteile einen festen Trägerstoff und eine Aromakomposition umfassen. Geeignete Aromakompositionen enthalten z.B. synthetische, natürliche oder naturidentische Aroma-, Riech- und Ge-schmacksstoffe, Reaktionsaromen, Raucharomen oder andere aromagebende Zuberei-tungen (z.B. Protein[teil]hydrolysate, bevorzugt Protein[teil]hydrolysate mit hohem Argi-nin-Gehalt, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die Aromenkompositionen oder deren Bestandteile geeignet, die einen röstigen, fleischigen (insbesondere Huhn, Fisch, Meerestiere, Rind, Schwein, Lamm, Schaf, Zie-ge), gemüsigen (insbesondere Tomate, Zwiebel, Knoblauch, Sellerie, Lauch, Pilze, Auberginen, Seetang), einen würzigen (insbesondere schwarzer und weißer Pfeffer, Kardamom, Muskat, Piment, Senf und Senf-Produkte), gebratenen, hefigen, gesotteen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Die Energiedichte eines oral konsumierbaren Produktes (insbesondere Lebensmittels, Futtermittels oder Arzneimittels) lässt sich senken, indem energiereiche Inhaltsstoffe des oral konsumierbaren Produktes gegen Ersatzstoffe (z.B. kalorienarme Verdickungsmittel statt Fette, kalorienarme oder kalorienfreie Süßstoffe statt üblicher Zucker) ausgetauscht werden. Der bereits oben diskutierte Nachteil, dass der Konsument ein oral konsumierbares Produkt (insbesondere ein Lebensmittel) mit reduzierter Energiedichte in größeren Mengen konsumiert und es dann im ungünstigsten Fall sogar zu einer erhöhten Aufnahme kalorisch relevanter Nahrungsmittelbestandteile kommt, wird durch die Verwendung des aromatischen Alkensäurederivats oder eines Gemischs von aromatischen Alkensäurederivate in oral konsumierbaren Lebensmitteln (insbesondere in Lebensmitteln) entgegengewirkt. In einem oral konsumierbaren Produkt enthaltenes aromatisches Alkensäurederivat oder eines Gemischs von aromatischen Alkensäurederivaten vermittelt ein vorzeitiges Gefühl von Sättigung und reduziert gleichzeitig den Appetit und die Resorpti-on von Lipiden. Zudem hat es einen positiven Einfluss auf die Stimmung des Konsumen-ten, was sich ebenfalls positiv auswirkt.

Vorzugsweise ist ein erfindungsgemäßes oral konsumierbares Produkt ausgewählt aus der Gruppe bestehend aus Süßwaren, vorzugsweise kalorienreduzierte bzw. kalorienfreie Süßwaren, vorzugsweise ausgewählt aus der Gruppe bestehend aus Müsliriegelprodukte, Fruchtgummi, Dragees, Hartkramellen und Kaugummi,
- nicht-alkoholische Getränke, vorzugsweise ausgewählt aus der Gruppe bestehend aus Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder -freie Limonaden, isotonische Getränke, Nektare, Obst- und Gemüsesäfte, Frucht- und Gemüsesaftzubereitungen,
- Instantgetränke, vorzugsweise ausgewählt aus der Gruppe bestehend aus Instant- (Grün-, Schwarz-, Rooibos-, Käuter-)Tee-Getränke,
- Getreideprodukte, vorzugsweise ausgewählt aus der Gruppe bestehend aus zuckerarmen und -freien Frühstückscerealien und Müsliriegeln,
- Milchprodukte, vorzugsweise ausgewählt aus der Gruppe bestehend aus fettreduzierten und fettfreien Milchgetränken, Joghurt, Kefir, Molke, Buttermilch und Eiscreme,
- Produkte aus Sojaprotein oder anderen Sojabohnen-Fraktionen, vorzugsweise ausgewählt aus der Gruppe bestehend aus Sojamilch, aus Sojamilch gefertigten Produkten, Getränken enthaltend isoliertes oder enzymatisch behandeltes Sojaportein, Sojamehl enthaltenden Getränken, Sojalecithin-haltigen Zubereitungen, aus Sojalecithin-haltigen Zubereitungen gefertigten Produkten und Mischungen mit Fruchtzubereitungen und ggf. Aromen,
- Süßstoffzubereitungen, -tabletten und -sachets
- Zuckerfreie Dragees,
- Eiscreme, mit oder ohne Bestandteile auf Basis von Milch, vorzugsweise zuckerfrei.

Bevorzugt enthält ein erfindungsgemäßes oral konsumierbares Produkt (insbesondere Lebensmittel, Futtermittel oder Arzneimittel) (a) einen, zwei oder mehrere Süßstoffe und/oder (b) ein, zwei oder mehrere Verdickungsmittel.

Der Begriff "Süßstoffe" bezeichnet hierbei Stoffe mit einer relativen Süßkraft von zumin-dest 25, bezogen auf die Süßkraft von Saccharose (welches somit die Süßkraft 1 hat). Vorzugsweise sind in einem erfindungsgemäßen oral konsumierbaren Produkt (insbesondere Lebensmittel, Futtermittel oder Arzneimittel) (a) einzusetzende Süßstoffe nichtkariogen und/oder besitzen einen Energiegehalt von maximal 5 kcal pro Gramm des oral konsumierbaren Produktes.

Vorteilhafte Süßstoffe in einem bevorzugten erfindungsgemäßen oral konsumierbaren Produkt (insbesondere Lebensmittel, Futtermittel oder Arzneimittel) sind ausgewählt aus den folgenden Gruppen (a1) und (a2):
(a1) natürlich vorkommende Süßstoffe, vorzugsweise ausgewählt aus der Gruppe bestehend aus
(a1-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physio-logisch akzeptablen Salzen dieser Aminosäuren und/oder Proteine, insbesondere den Natrium-, Kalium-, Calcium oder Ammoniumsalzen;
(a1-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Re-baudiosid D, Rebaudiosid E, Rebaudiosid F, Rebaudiosid G, Rebaudiosid H, Rebaudiosid M, Rebaudiosid X, Dul-cosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A15, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium- oder Ammoniumsalzen;
(a1-3) Extrakte oder angereicherte Fraktionen der Extrakte, ausgewählt aus der Gruppe bestehend aus Thaumatococcus-Extrakten (Katemfestaude), Extrakten aus Stevia ssp. (insbesondere Stevia rebaudiana), Swingle-Extrakten (Momordica bzw. Siratia grosvenorii, Luo-Han-Guo), Extrakten aus Glycerrhyzia ssp. (insbesondere Gly-cerrhyzia glabra), Extrakten aus Rubus ssp. (insbesondere Rubus suavissimus), Citrus-Extrakten und Extrakten aus Lippia dulcis;
(a2) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen des Acesulfam, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

Vorteilhafte Verdickungsmittel in einem bevorzugten erfindungsgemäßen oral konsumier-baren Produkt (insbesondere Lebensmittel, Futtermittel oder Arzneimittel) sind ausgewählt aus der Gruppe bestehend aus: vernetzte Polyacrylsäuren und deren Derivate, Polysaccharide und deren Derivate, wie Xanthangummi, Agar-Agar, Alginate oder Tylo-sen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.

Erfindungsgemäß bevorzugt ist ein oral konsumierbares Produkt (insbesondere Lebens-mittel oder Futtermittel), das durch Milchsäurebakterien verdickte Milch und/oder durch Milchsäurebakterien verdickte Sahne enthält und vorzugsweise ausgewählt ist aus der Gruppe bestehend aus oral konsumierbaren Produkten mit einem Fettgehalt von 4,0 Gew.-% oder weniger, bevorzugt von 1,5 Gew.-% oder weniger, besonders bevorzugt 0,5 Gew.-% oder weniger, jeweils bezogen auf das Gesamtgewicht des oral konsumierbaren Produktes, und/oder ausgewählt ist aus der Gruppe bestehend aus Joghurt, Kefir und Quark.

Vorzugsweise enthält das erfindungsgemäße oral konsumierbare Produkt (insbesondere Lebensmittel oder Futtermittel), das durch Milchsäurebakterien verdickte Milch und/oder durch Milchsäurebakterien verdickte Sahne enthält, einen Energiegehalt von nicht mehr als 150 kcal/100g des oral konsumierbaren Produktes, bevorzugt nicht mehr als 100 kcal/100g, besonders bevorzugt nicht mehr als 75 kcal/100g, besonders bevorzugt nicht mehr als 50 kcal/100g.

Ein bevorzugtes erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, umfasst zusätzlich Früchte und/oder Fruchtzubereitungen.

Besonders bevorzugt ist ein erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, wobei das oral konsumierbare Produkt (i) Zucker und/oder (ii) Verdickungsmittel und/oder (iii) Geliermittel und/oder (iv) Süßungsmittel und/oder (v) Aromen und/oder (vi) Konservierungsstoffe enthält.

"Zucker" ist im Rahmen des vorliegenden Textes (sofern nicht anders angegeben oder aus dem Kontext anders ersichtlich) der Sammelbegriff für alle süß schmeckenden Saccharide (Einfach- und Doppelzucker).

Vorteilhafterweise ist ein erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, ein oral konsumierbares Produkt, das ein Probiotikum enthält, wobei das Probiotikum vorzugsweise ausgewählt ist aus der Gruppe bestehend aus Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) und Lactobacillus rham-nosus (ATCC 53013).

Besonders bevorzugt ist ein erfindungsgemäßes oral konsumierbares Produkt (insbesondere Lebensmittel, Futtermittel oder Arzneimittel), das ein Kaugummi ist und eine Kaugummibase enthält. Die Kaugummibase ist dabei vorzugsweise ausgewählt aus der Gruppe bestehend aus "chewing gum" - oder "bubble gum" - Basen. Letztere sind weicher, damit sich damit auch Kaugummiblasen bilden lassen. Erfindungsgemäß bevorzugte Kaugummibasen umfassen neben traditionell eingesetzten natürlichen Harzen oder dem Naturlatex Chicle Elastomere wie Polyvinylacetate (PVA), Polyethylene, (nieder- oder mittel-molekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispiels-weise in EP 0 242 325, US 4,518,615, US 5,093,136, US 5,266,336, US 5,601,858 oder US 6,986,709 beschrieben. Daneben umfassen erfindungsgemäß bevorzugt einzusetzende Kaugummibasen vorzugsweise weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbo-nat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearin-säure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Erfindungsgemäße Kaugummis (insbesondere wie vorstehend offenbart) umfassen vorzugsweise Bestandteile wie Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropion-säure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist unter anderem die Kombination der Aromen mit weiteren Stoffen, die kühlende, wärmende und/oder mundwässerndernde Eigenschaften aufweisen.

Besonders bevorzugt ist ein erfindungsgemäßes oral konsumierbares Produkt (insbesondere Lebensmittel, Futtermittel oder Arzneimittel), wobei das oral konsumierbare Produkt ein Getränk ist, wobei das Getränk vorzugsweise einen Zuckergehalt von 30 g/100 mL Getränk oder weniger, bevorzugt von 15 g/100 mL oder weniger, besonders bevorzugt 5 g/100 mL oder weniger aufweist, besonders bevorzugt kein Zucker enthält
und/oder
wobei das Getränk kein Ethanol oder maximal 0,1 Volumenprozent Ethanol enthält, bezogen auf das Volumen des Getränkes.

Im Rahmen der vorliegenden Erfindung sind erfindungsgemäße oral konsumierbare Produkte weniger bevorzugt, bei denen es sich um Ethanol enthaltende Getränke handelt.

Kein Ethanol bedeutet in der vorliegenden Erfindung, dass kein Ethanol zugesetzt wird und dass die Zubereitung weniger als 0,1 Vol.-%, bevorzugt weniger als 0,01 Vol.-% und besonders bevorzugt keine messbare Menge an Ethanol enthält.

Besonders bevorzugt sind erfindungsgemäße oral konsumierbare Produkte (vorzugsweise Lebensmittel, Futtermittel oder Arzneimittel), wobei es sich um ein kohlensäurehaltiges Getränk oder um ein kohlensäurefreies Getränk handelt.

Weitere Aspekte der vorliegenden Erfindung ergeben sich aus den nachfolgenden Beispielen und den beigefügten Ansprüchen.

### BEISPIELE

### Herstellungsbeispiele 1 bis 4

### Verbindung 1: 2E-5-(1,3-Benzodioxol-5-yl)-N-isobutyl-pent-2-enamid (Dihydropiperlinguminin)

Verbindung 1 wurde aus einem angereicherten ethanolisch-wässrigen Extrakt von *Macropiper excelsum* durch präparative HPLC isoliert. Die spektroskopischen Daten finden sich in Tabelle 1.

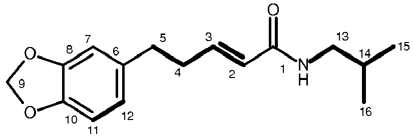

**Tabelle 1**

| Spektroskopische Daten | | | | |
|---|---|---|---|---|
| **Position** | **¹H NMR 400 MHz,CDCl₃** | | **¹C NMR 100 MHz,CDCl₃** | |
| | ***δ*_{H} (ppm), mult (*J* in Hz)** | | ***δ*_{c} (ppm)** | |
| 1 | | | 165.8, C | |
| 2 | 5.77, dt (1.5, 15.2) | | 124.2, CH | |
| 3 | 6.84, dt (6.9, 15.2) | | 143.3, CH | |
| 4 | 2.45, m | | 34.1, CH2 | |
| 5 | 2.69, m | | 34.4, CH2 | |
| 6 | | | 134.9, C | |
| 7 | 6.67, dd (0.4, 1.7) | | 108.8, CH | |
| 8 | | | 147.6, C | |
| 9 | 5.92, s | | 100.8, CH2 | |
| 10 | | | 145.8, C | |
| 11 | 6.73, dd (0.4, 7.8) | | 108.2, CH | |
| 12 | 6.62, ddt (0.6, 1.7, 7.9) | | 121.1, CH | |
| 13 | 3.15, dd (6.1, 6.9) | | 46.8, CH2 | |
| 14 | 1.79, thept (6.7) | | 28.6, CH | |
| 15/16 | 0.92, d (6.7) | | 20.1, CH3 | |

| **HR-MS (calc)** | **HR-MS** | **[*M*+H]⁺** | ***m*/*z*** | **UV λₘₐₓ (nm)** |
|---|---|---|---|---|
| 276.1594 | 276.1588 | C16H22NO3 | 135.0466 | 200, 215, 285 |

### Verbindung 2:

### 2E,4E-7-(1,3-Benzodioxol-5-yl)-N-isobutyl-hepta-2,4-dienamid (Ching-chengenamide A)

Verbindung 2 wurde aus einem angereicherten ethanolisch-wässrigen Extrakt von Macropiper excelsum durch präparative HPLC isoliert. Die spektroskopischen Daten finden sich in **Tabelle 2.**

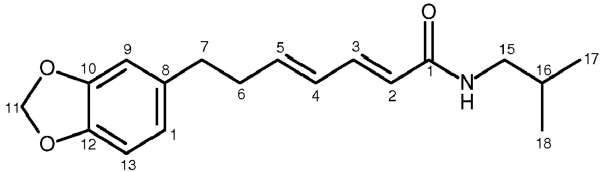

**Tabelle 2**

| Spektroskopische Daten | | | | | |
|---|---|---|---|---|---|
| **Position** | ¹**H NMR 400 MHz, CD₃OD** | | | **¹³C NMR 100 MHz, CD₃OD** | |
| | ***δ*_{H} (ppm), mult (*J* in Hz)** | | | ***δ*_{c} (ppm)** | |
| 1 | | | | 169.1, C | |
| 2 | 5.91, dt (0.5, 15.2) | | | 123.4, CH | |
| 3 | 7.08, dd (10.5, 15.2) | | | 142.0, CH | |
| 4 | 6.18, ddq (0.7, 1.1, 10.5, 15.2) | | | 130.3, CH | |
| 5 | 6.08, dt (6.7, 15.2) | | | 142.8, CH | |
| 6 | 2.43, q (6.8) | | | 36.1, CH2 | |
| 7 | 2.66, t (7.6) | | | 36.0, CH2 | |
| 8 | | | | 136.6, C | |
| 9 | 6.69, p (0.5, 1.8) | | | 109.8, CH | |
| 10 | | | | 149.0, C | |
| 11 | 5.88, s | | | 102.0, CH2 | |
| 12 | | | | 147.2, C | |
| 13 | 6.70, d (7.9) | | | 109.0, CH | |
| 14 | 6.63, ddt (0.5, 1.8, 7.9) | | | 122.3, CH | |
| 15 | 3.06, d (6.9) | | | 48.0, CH2 | |
| 16 | 1.79, thept (6.9) | | | 29.8, CH | |
| 17/18 | 0.91, d (6.7) | | | 20.5, CH3 | |
| | | | | | |

| **HR-MS (calc)** | **HR-MS** | **[*M*+H]⁺** | ***m*/*z*** | | **UV λₘₐₓ (nm)** |
|---|---|---|---|---|---|
| 302.1751 | 302.1737 | C18H24NO3 | 135.0454 | | 200, 220, 260 |

### Verbindung 3:

### 2E,4E-7-(1,3-Benzodioxol-5-yl)-1-(1-piperidyl)hepta-2,4-dien-1-on (Piperdardin)

Verbindung 3 wurde aus einem angereicherten ethanolisch-wässrigen Extrakt von *Macropiper excelsum* durch präparative HPLC isoliert. Die spektroskopischen Daten finden sich in Tabelle 3.

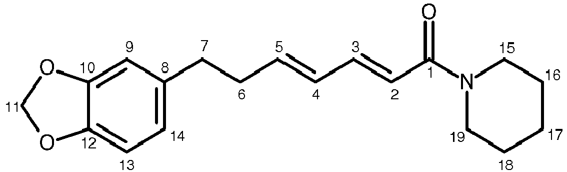

**Tabelle 3**

| Spektroskopische Daten | | | | | | |
|---|---|---|---|---|---|---|
| **Position** | **¹H NMR 400 MHz, CD₃OD** | | | | **¹³C NMR 100 MHz, CD₃OD** | |
| | ***δ*_{c} (ppm), mult *δ*_{H} (*J* in Hz)** | | | | ***δ*_{c} (ppm)** | |
| 1 | | | | | 167.8, C | |
| 2 | 6.43, d (14.8) | | | | 119.9, CH | |
| 3 | 7.12, dd (10.8, 14.8) | | | | 144.4, CH | |
| 4 | 6.26, dd (10.8, 15.2) | | | | 130.8, CH | |
| 5 | 6.09, dt (6.9, 15.2) | | | | 142.9, CH | |
| 6 | 2.43, q (7.3) | | | | 36.1, CH2 | |
| 7 | 2.66, t (7.5) | | | | 36.0, CH2 | |
| 8 | | | | | 136.5, C | |
| 9 | 6.69, d (1.7) | | | | 109.8, CH | |
| 10 | | | | | 149.0, C | |
| 11 | 5.88, s | | | | 102.0, CH2 | |
| 12 | | | | | 147.2, C | |
| 13 | 6.70, d (7.9) | | | | 109.0, CH | |
| 14 | 6.63, dd (1.7, 7.8) | | | | 122.3, CH | |
| 15 | 3.58, m | | | | 44.5, 48.1 CH2 | |
| 16 | 1.57, m | | | | 26.9, 27.8, CH2 | |
| 17 | 1.69, p (5.6) | | | | 25.5, CH2 | |
| 18 | 1.57, m | | | | 26.8, 27.8, CH2 | |
| 19 | 3.58, m | | | | 44.5, 48.1 CH2 | |
| | | | | | | |

| **HR-MS (calc)** | | **HR-MS** | **[*M*+H]⁺** | ***m*/*z*** | | **UV λₘₐₓ (nm)** |
|---|---|---|---|---|---|---|
| 314.1751 | | 314.1752 | C19H24NO3 | 135.0451 | | 200,265 |

### Verbindung 4:

### 2E,4E-Methyl-7-(1,3-benzodioxol-5-yl)hepta-2,4-dienoat

Verbindung 4 wurde aus einem angereicherten ethanolisch-wässrigen Extrakt von *Macropiper excelsum* durch präparative HPLC isoliert. Die spektroskopischen Daten finden sich in **Tabelle 4.**

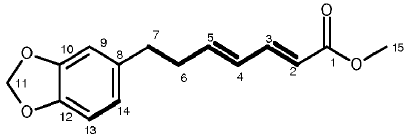

**Tabelle 4**

| Spektroskopische Daten | | | | | | |
|---|---|---|---|---|---|---|
| **Position** | **¹³C NMR 100 MHz, CD₃OD** | | | **¹H NMR 400 MHz, CD₃OD** | | |
| | ***δ*_{H} (ppm)** | | | ***δ*_{H} (ppm), mult (*J* in Hz)** | | |
| 1 | 169.4, C | | | | | |
| 2 | 119.9, CH | | | 5.81, d (15.4) | | |
| 3 | 146.7, CH | | | 7.23, dd (9.9, 15.4) | | |
| 4 | 130.1, CH | | | 6.24, dd (9.9, 15.0) | | |
| 5 | 145.1, CH | | | 6.18, dt (6.4, 15.0) | | |
| 6 | 35.8, CH2 | | | 2.45, m | | |
| 7 | 36.2, CH2 | | | 2.67, t (7.6) | | |
| 8 | 136.4, C | | | | | |
| 9 | 109.8, CH | | | 6.69, d (1.7) | | |
| 10 | 149.1, C | | | | | |
| 11 | 102.0, CH2 | | | 5.88, s | | |
| 12 | 147.2, C | | | | | |
| 13 | 109.0, CH | | | 6.70, d (8.0) | | |
| 14 | 122.3, CH | | | 6.63, dd (1.7, 7.8) | | |
| 15 | 52.0, CH3 | | | 3.71, s | | |
| | | | | | | |

| **HR-MS (calc)** | | **HR-MS** | **[*M*+H]⁺** | | ***m*/*z*** | **UV λₘₐₓ (nm)** |
|---|---|---|---|---|---|---|
| 261.1121 | | 261.1112 | C15H17O4 | | 128.0628, 135.0451 | 200,260 |

### Beispiel 5:

### Bestimmung der Serotonin-Ausschüttung in Neuroblastomazellen durch Verbindungen 3 und 4

Als Zellmodell für die Ausschüttung des Neurotransmitters Serotonin werden humane Neuroblastomazellen (SH-SY5Y, ATCC Nummer CRL-2266) verwendet. Die Kultivierung erfolgt bei 37 °C und 5 % CO2-Gehalt mit einer Mischung, die zu gleichen Teilen aus Eagle's Minimum Essential Medium (MEM) und F12 Medium (jeweils mit 10 % FBS und 1 % Penicillin / Streptomycin) besteht. Serotoninfreisetzung werden die Zellen mit Trypsin geerntet und nach einer Vitalitätsprüfung durch Trypanblaufärbung in definierter Zellzahl in 35 mm Zellkulturschalen ausgesät.

### Beispiel 6:

### Freisetzung von Serotonin in einem experimentellen Zellsystem mit Verbindungen 1 bis 4

Nach 3-minütiger Stimulierung von 1,25 * 106 humanen Neuroblastomazellen (SH SY5Y) mit 300 µL Krebs-Ringer-HEPES Puffer, 0,1 % Ascorbinsäure, pH 6,2, mit oder ohne Zusatz eine der Verbindungen 3 und 4, wobei bei Zusatz der Verbindung in Konzentrationen von 0,01 µM, 0,1 µM 1 µM und 10 µM im Krebs-Ringer-HEPES Puffer eingestellt wird), wird der Serotoningehalt mit Hilfe eines enzymbasierten Nachweisverfahrens (Serotonin-ELISA sensitiv, DLD Diagnostica, Hamburg, Deutschland) ermittelt. Die Zellen werden mit einem natriumlaurylsarcosinathaltigem Puffer lysiert und der DNA-Gehalt mit Hilfe einer Nano-Quant-Platte (Tecan, Mennendorf, Schweiz) zur Normalisierung der Serotoninfreisetzung ermittelt.

**Tabelle 5**

| Serotonin-Freisetzung SH-SY5Y-Zellen nach Stimulierung mit verschiedenen Konzentrationen der Verbindunger 3 und 4. | | |
|---|---|---|
| **Testsubstanz** | **Serotoninfreisetzung [% der Kontrelle]** | **Standardabweichung [%]** |
| EtOH Kontrolle (Verbindung 3) | 100 | 34.3 |
| Verbindung 3, 0,01 µM | 161 | 69.8 |
| Verbindung 3, 0,1 µM | 141 | 47.6 |
| Verbindung 3, 1 µM | 283 | 79.8 |
| Verbindung 3, 10 µM | 226 | 125 |
| Verbindung 4, 0,01 µM | 129 | 82.1 |
| Verbindung 4, 0,1 µM | 123 | 72.1 |
| Verbindung 4, 1 µM | 239 | 113 |
| Verbindung 4, 10 µM | 171 | 29.7 |

**Tabelle 5** zeigt den Einfluss von Verbindungen 3 und 4 auf die Serotoninfreisetzung in SH-SY5Y Zellen. Eine Konzentration von 1 µM Verbindung 3 zeigt 280%ige, von 1 µM Verbindung 4 eine 240 %ige Steigerung der Serotonin-Freisetzung gegenüber der Kontrolle.

### ANWENDUNGSBEISPIELE

### Anwendungsbeispiel 7 : Erfrischungsgetränke (zuckerhaltig, kalorienreduziert, kalorienfrei)

Die Zutaten wurden in der angegebenen Reihenfolge gemischt und mit Wasser auf 100 % aufgefüllt. Die Mischungen werden in Glasflaschen gefüllt und karbonisiert. Die Zusammensetzung ist in **Tabelle A** wiedergegeben.

**Tabelle A**

| Zusammensetzung Erfrischungsgetränk | | | | | | | |
|---|---|---|---|---|---|---|---|
| **Inhalstoff** | **Einsatz in Gew.-%** | | | | | | |
| | **A** | **B** | **C** | **D** | **E** | **F** | **G** |
| Zucker (Sucrose) | 10 | 10 | 7 | - | - | 8 | 7 |
| Glucose/Fructose-Sirup aus Mais, enthaltend 55 Gew.-% Fructose | - | - | - | - | 10 | - | - |
| Rebaudiosid A 95 % | - | - | 0,02 | 0,05 | - | - | - |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Coffein | - | - | 0,01 | - | - | - | - |
| Zitronenaroma | 0,1 | 0,05 | - | 0,1 | 0,1 | 0,1 | 0,1 |
| Limonenaroma | - | 0,05 | - | - | - | - | - |
| Getränke-Emulsion Typ "Cola" | - | - | 0,05 | - | - | - | - |
| Phloretin | - | - | 0,002 | 0,003 | - | 0,002 | 0,001 |
| Hesperetin | - | - | 0,001 | 0,002 | - | - | 0,002 |
| Extrakt aus Rubus suavissimus, enthaltend 5 Gew.-% Rubusosid bez. auf das Gesamtgewicht des Extraktes | - | - | - | - | - | 0,01 | - |
| Homoeriodictyol-Natriumsalz | - | - | 0,005 | - | 0,005 | - | 0,005 |
| trans-Pellitorin | - | - | - | - | - | 0,0001 | 0,0001 |
| Verbindung 1 | - | - | - | 0,0005 | - | 0,0002 | 0,0001 |
| Verbindung 2 | - | - | 0,0002 | - | - | - | 0,0001 |
| Verbindung 3 | 0,0002 | 0,0001 | - | 0,0005 | 0,0001 | 0,0002 | 0,0001 |
| Verbindung 4 | - | 0,0001 | 0,0002 | - | - | - | 0,0001 |
| Wasser | Ad 100 | | | | | | |

### Anwendungsbeispiel 8: Verwendung in einem Kaugummi

Teile I bis IV werden gemischt und intensiv geknetet. Die Rohmasse kann z.B. in Form von dünnen Streifen zu verzehrsfertigen Kaugummis verarbeitet werden. Die Zusammensetzung ist in **Tabelle B** wiedergegeben.

**Tabelle B**

| Zusammensetzung Kaugummi | | | | | | |
|---|---|---|---|---|---|---|
| **Teil** | **Inhaltsstoff** | **Einsatz in GEW.%** | | | | |
| | | **A** | **B** | **C** | **D** | **E** |
| I | Kaugummibase, Company "Jagum T" | 30,495 | 30,495 | 30,4948 | 30,495 | 30,495 |
| II | Sorbit, pulverisiert | 39,00 | 39,00 | 39,00 | 39,00 | 39,00 |
| | Isomalt^{®} (Palatinit GmbH) | 9,50 | 9,50 | 9,50 | 9,50 | 9,50 |
| | Xylit | 2,00 | 2,00 | 2,00 | 2,00 | 2,00 |
| | Mannit | 3,00 | 3,00 | 3,00 | 3,00 | 3,00 |
| | Aspartam^{®} | 0,10 | 0,10 | 0,10 | 0,10 | 0,10 |
| | Acesulfam^{®} K | 0,10 | 0,10 | 0,10 | 0,10 | 0,10 |
| | Emulgum^{®} (Colloides Naturels, Inc.) | 0,30 | 0,30 | 0,30 | 0,30 | 0,30 |
| III | Sorbitol, 70% | 14,00 | 14,00 | 14,00 | 14,00 | 14,00 |
| | Glycerin | 1,00 | 1,00 | 1,00 | 1,00 | 1,00 |
| IV | Pfefferminzaroma | 0,5 | 0,5 | - | 0,5 | 0,5 |
| | Zimt-Frucht-Aroma | - | - | 0,5 | - | - |
| | trans-Pellitorin | - | - | 0,0002 | - | - |
| | Nonivamid | - | - | - | 0,00001 | 0,00001 |
| | Verbindung 1 | - | - | 0,0025 | - | - |
| | Verbindung 2 | - | - | 0,0025 | - | 0,0050 |
| | Verbindung 3 | 0,0050 | - | 0,0025 | 0,0025 | - |
| | Verbindung 4 | - | 0,0050 | 0,0025 | 0,0025 | 0,0050 |

### Anwendungsbeispiel 9: Verwendung in Hartkaramellen

Palatinit bzw. der Zucker wurden ggfs. nach Zugabe der Zitronensäure mit Wasser gemischt und die Mischung bei 165 °C aufgeschmolzen und anschließend auf 115°C abgekühlt. Das Aroma und die anderen Bestandteile wurden zugegeben und nach dem Durchmischen in Formen gegossen, nach dem Erstarren aus den Formen entfernt und anschließend einzeln verpackt. Die Zusammensetzung ist in **Tabelle C** wiedergegeben.

**Tabelle C**

| Zusammensetzung Hartkaramellen | | | | | |
|---|---|---|---|---|---|
| **Inhaltsstoffe** | **Gehalt (Gew.%)** | | | | |
| | **A** | **A** | **C** | **D** | **E** |
| Zucker | 74,50 | - | - | - | - |
| Palatinit, Typ M | - | 74,00 | 75,50 | 75,00 | 75,00 |
| Zitronensäure | 0,5 | 1,0 | 0,5 | - | - |
| Farbstoff gelb | - | 0,01 | - | - | - |
| Farbstoff rot | - | - | 0,01 | - | - |
| Farbstoff blau | 0,01 | - | - | 0,01 | 0,01 |
| Pfefferminzaroma | 0,1 | - | - | 0,1 | 0,1 |
| Zitronenaroma | - | 0,1 | - | - | - |
| Rotfruchtaroma | - | - | 0,1 | - | - |
| Rebaudiosid A 98 % | - | 0,040 | - | 0,040 | 0,040 |
| Balansin A nach WO2012164062 | - | 0,005 | 0,010 | 0,005 | - |
| Hesperetin | - | 0,001 | - | 0,001 | 0,001 |
| Phloretin | - | 0,002 | - | - | - |
| trans-Pellitorin | - | - | 0,00002 | 0,00002 | - |
| Nonivamid | - | - | 0,000005 | - | 0,00001 |
| Verbindung 1 | 0,0002 | 0,0001 | - | - | - |
| Verbindung 2 | - | 0,0001 | - | - | |
| Verbindung 3 | 0,0002 | 0,0001 | - | 0,0001 | 0,0002 |
| Verbindung 4 | - | 0,0001 | 0,0002 | 0,0001 | - |
| Wasser | ad 100 | ad 100 | ad 100 | ad 100 | ad 100 |

### Anwendungsbeispiel 10: Fettarme Joghurts

Die Inhaltsstoffe wurden gemischt und auf 5°C gekühlt. Die Zusammensetzung ist in **Tabelle D** wiedergegeben.

**Tabelle D**

| Zusammensetzung Joghurts | | | | |
|---|---|---|---|---|
| **Inhaltsstoffe** | **Gehat in Gew. %** | | | |
| | **A** | **B** | **C** | **D** |
| Sucrose | 10 | 8 | 6 | - |
| Rebaudiosid A 98 % | - | - | - | 0,050 |
| Extrakt aus Rubus suavissimus, enthaltend 5 Gew.-% Rubusosid bezogen auf das Gesamtgewicht des Extraktes, z.B. von PlantExtrakt | - | 0,010 | 0,010 | - |
| Hesperetin | - | 0,001 | 0,001 | 0,002 |
| Phloretin | - | - | 0,002 | 0,002 |
| Homoeriodictyol-Natriumsalz | - | - | - | 0,005 |
| Verbindung 1 | - | - | - | 0,0002 |
| Verbindung 2 | - | - | 0,00025 | - |
| Verbindung 3 | 0,0005 | 0,00025 | - | 0,0002 |
| Verbindung 4 | - | 0,00025 | 0,0004 | 0,0002 |
| Nonivamid | - | - | - | 0,00001 |
| Joghurt, 0,1 % Fett | zu 100 % auffüllen | | | |

### Anwendungsbeispiel 11: Fruchtgummis

**Tabelle E**

| Zusammensetzung Fruchtgummis | | | | |
|---|---|---|---|---|
| | **Zybereitung (Angaben als Gew.%)** | | | |
| **Inhaltsstoff** | **A** | **B** | **C** | **D** |
| Saccharose | 34,50 | 8,20 | 8,20 | 34,50 |
| Glucosesirup, DE 40 | 31,89 | 30,09 | 30,09 | 31,89 |
| Iso Syrup C* Tru Sweet 01750 (Cerestar GmbH) | 1,50 | 2,10 | 2,10 | 1,50 |
| Gelatine 240 Bloom | 8,20 | 9,40 | 9,40 | 8,20 |
| Polydextrose (Litesse^{®} Ultra, Danisco Cultor GmbH) | - | 24,40 | 24,40 | - |
| Gelber und roter Farbstoff | 0,01 | 0,01 | 0,01 | 0,01 |
| Citronensäure | 0,20 | | | 0,20 |
| Kirscharoma, enthaltend 1 Gew.-% Hesperetin 2 und 0,3 Gew.-% Phloretin bezogen auf das Aroma | - | 0,10 | 0,10 | - |
| trans-Pellitorin | - | - | 0,00002 | - |
| Nonivamid | - | - | 0,000005 | - |
| Verbindung 1 | - | 0,0001 | 0,0002 | - |
| Verbindung 2 | - | - | - | 0,0002 |
| Verbindung 3 | 0,0002 | 0,0001 | - | 0,0002 |
| Verbindung 4 | - | 0,0001 | 0,0002 | - |

Polydextrose ist ein nicht süß schmeckendes Polysaccharid mit niedrigem Brennwert.

### Anwendungsbeispiel 12: Fruchtsäfte und Fruchtsaftgetränke

**Tabelle F**

| Zusammensetzung Fruchtsaft | | | | | | | |
|---|---|---|---|---|---|---|---|
| **Inhaltsstoff** | **Einsatz in Gew.-%** | | | | | | |
| | **A** | **B** | **C** | **D** | **E** | **F** | **G** |
| Apfelsaft | ad 100 | - | - | - | ad 100 | - | ad 100 |
| Apfelsaftkonzentrat 10fach | - | 10 | - | 10 | - | 10 | - |
| Recovery-Aroma aus Apfelsaftkonzentrat | - | 0,1 | - | 0,1 | - | 0,1 | - |
| Orangensaftkonzentrat 20fach | - | - | 5 | - | - | - | - |
| Recovery-Aroma aus Orangensaftkonzentrat | - | - | 0,05 | - | - | - | - |
| Wasser | - | - | ad 100 | ad 100 | - | ad 100 | - |
| Ascorbinsäure | 0,05 | 0,05 | 0,05 | - | - | - | - |
| trans-Pellitorin | - | - | - | - | - | 0,0001 | - |
| Nonivamid | - | - | - | - | 0,00005 | 0,00005 | 0,00005 |
| Verbindung 1 | - | - | - | 0,0002 | 0,0001 | - | 0,0002 |
| Verbindung 2 | - | - | 0,0002 | - | - | 0,0001 | - |
| Verbindung 3 | 0,0002 | 0,0001 | - | 0,0002 | 0,0001 | 0,0002 | 0,0002 |
| Verbindung 4 | - | 0,0001 | 0,0002 | - | - | - | - |

### Anwendungsbeispiel 13: Milchmixgetränk auf Trockenbasis

Die Zutaten werden gemischt und unter Schutzgas oder im Vakuumbeutel abgepackt. Zur Verwendung wird das Pulver in 100 ml Wasser (Zimmertemperatur) aufgerührt und dann verzehrt. Die Zusammensetzung ist in **Tabelle G** wiedergegeben.

**Tabelle G**

| Zusammensetzung Milchmixgetränk | | | | | | | |
|---|---|---|---|---|---|---|---|
| **Inhaltsstoff** | **Einsaz in Gew.-%** | | | | | | |
| | **A** | **B** | **C** | **D** | **E** | **F** | **G** |
| Zucker, fein | 5 | 5 | 5 | 5 | 5 | 5 | 5 |
| Nonivamid, 0,1%ig in Alkohol | - | - | 0,03 | 0,03 | - | 0,015 | - |
| trans-Pellitorin, 1 %ig in 1,2-Propylenglycol/Diethylmalonat | - | 0,03 | - | 0,03 | - | - | 0,01 |
| Verbindung 3, 1 %ig in Alkohol | 0,03 | - | 0,03 | 0,03 | 0,05 | 0,03 | 0,03 |
| Verbindung 4, 1 %ig in Alkohol | - | 0,03 | 0,03 | 0,01 | 0,02 | - | 0,03 |
| Aroma Typ Cappuccino sprühgetrocknet | 0,2 | 0,2 | 0,2 | 0,2 | - | - | - |
| Aroma Typ Erdbeer sprühgetrocknet | - | - | - | - | 0,1 | 0,1 | 0,1 |
| Rote Bete Farbpulver sprühgetrocknet | - | - | - | - | 0,1 | 0,08 | 0,1 |
| Zuckercouleur | 0,15 | 0,15 | 0,15 | 0,15 | - | - | - |
| Xanthan | 0,1 | 0,1 | 0,1 | 0,1 | 0,1 | 0,1 | 0,1 |
| Magermilchpulver | 10 | 10 | 10 | - | 10 | - | - |
| Milchpulver lactosereduziert (Fa. Omira, Ravensburg) | - | - | - | - | - | 10 | 10 |
| Lupinenproteinpulver | - | - | - | 5 | - | - | - |

## Patentansprüche

1. Aromatische Alkensäurederivate der Formel I 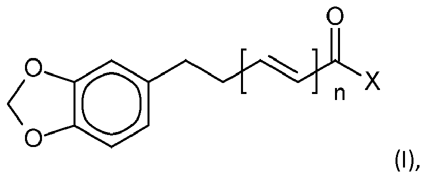 zur Anwendung in einem therapeutischen Verfahren
(i) zur Reduzierung des Appetits,
und/oder
(ii) zur Vermittlung eines Gefühls von Sättigung,
und/oder
(iii) als Stimmungsaufheller,
wobei
n = 1, 2 bedeutet, wobei die resultierende(n) Doppelbindung(en) unabhängig voneinander als E-oder Z-Konfiguration vorliegen,
X = - OR¹ oder - NR²R³, mit
R¹ = Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
oder
R² und R³ zusammen einen gesättigten oder ungesättigten carbocyclischen Ring ausbilden, ausgewählt aus der Gruppe bestehend aus: -CH₂-CH₂-, -CH₂-CH₂-CH₂-, -CH₂-CH₂-CH₂-CH₂-, -CH=CH-CH=CH-, -CH₂-CH₂-CH₂-CH₂-CH₂-, -CH=CH-CH₂-CH₂-CH₂-,-CH₂-CH=CH-CH₂-CH₂-, -CH=CH-CH=CH-CH₂- oder -CH=CH-CH=CH-CH=.

2. Nicht-therapeutische Verwendung von aromatischen Alkensäurederivate der Formel I 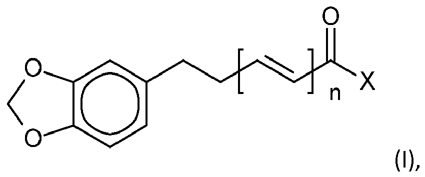
(i) zur Reduzierung des Appetits,
und/oder
(ii) zur Vermittlung eines Gefühls von Sättigung,
und/oder
(iii) als Stimmungsaufheller,
wobei
n = 1, 2 bedeutet, wobei die resultierende(n) Doppelbindung(en) unabhängig voneinander als E-oder Z-Konfiguration vorliegen,
X = - OR¹ oder - NR²R³, mit
R¹ = Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
und wobei
R², R³ unabhängig voneinander ausgewählt ist aus der Gruppe bestehend aus: Wasser-stoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
oder
R² und R³ zusammen einen gesättigten oder ungesättigten carbocyclischen Ring ausbilden, ausgewählt aus der Gruppe bestehend aus: -CH2-CH2-, -CH2-CH2-CH2-,-CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2- oder -CH=CH-CH=CH-CH=.

3. Aromatische Alkensäurederivate zur Anwendung oder Verwendung nach Anspruch 1 oder 2,
wobei
n= 1, 2, wobei die resultierende(n) Doppelbindung(en) zu mehr als 50% in E-Konfiguration vorliegt bzw. vorliegen,
X = - OR¹ oder -N^{R}2R³, mit
R¹= Methyl, Ethyl
R² = Wasserstoff,
R³ = 2-Methylpropyl, 2-Methyl-1-butyl,
oder
R² und R³ bilden zusammen einen gesättigten carbocyclischen Ring bestehend aus -CH2-CH2-CH2-CH2-CH2-, aus.

4. Aromatische Alkensäurederivate zur Anwendung oder Verwendung nach einem der vorhergehenden Ansprüche 1-3, wobei die aromatischen Alkensäurederivate ausgewählt sind aus der Gruppe bestehend aus:
5-((1,3-Benzodioxol-5-yl)-N-isobutyl-pent-2-enamid (Verbindung 1),
7-((1,3-Benzodioxol-5-yl)-N-isobutyl-hepta-2,4-dienamid (Verbindung 2),
7-((1,3-Benzodioxol-5-yl)-1-(1-piperidyl)hepta-2,4-dien-1-on (Verbindung 3),
Methyl-7-(1,3-benzodioxol-5-yl)hepta-2,4-dienoat (Verbindung 4).

5. Aromatische Alkensäurederivate zur Anwendung oder Verwendung nach einem der vorhergehenden Ansprüche 1-4, **dadurch gekennzeichnet, dass** die aromatischen Alkensäurederivate zu mehr als 50 % in der E-Konfiguration vorliegen.

6. Verfahren
(i) zur nicht-therapeutischen Reduzierung des Appetits,
und/oder
(ii) zur nicht-therapeutischen Vermittlung eines Gefühls von Sättigung,
und/oder
(iii) zur Aufhellung der Stimmung,
mit folgendem Schritt:
- Verabreichen einer (a) den Appetit reduzierenden und/oder (b) ein Gefühl von Sättigung bewirkenden und/oder (c) einer stimmungsaufhellenden Menge von mindestens einer aromatischen Alkensäurederivatverbindung der Formel I 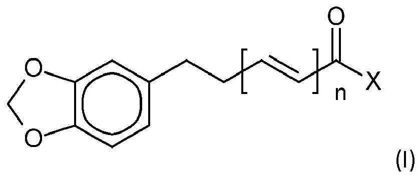 wobei
n = 1, 2 bedeutet, wobei die resultierende(n) Doppelbindung(en) unabhängig voneinander als E-oder Z-Konfiguration vorliegen,
X=-OR¹ oder -NR²R³, mit
R¹= Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
oder
R² und R³ zusammen einen gesättigten oder ungesättigten carbocyclischen Ring ausbilden ausgewählt aus der Gruppe bestehend aus: -CH₂-CH₂-, -CH₂-CH₂-CH₂-, -CH₂-CH₂-CH₂-CH₂-, -CH=CH-CH=CH-, -CH₂-CH₂-CH₂-CH₂-CH₂-, -CH=CH-CH₂-CH₂-CH₂-,-CH₂-CH=CH-CH₂-CH₂-, -CH=CH-CH=CH-CH₂- oder -CH=CH-CH=CH-CH=.

7. Zubereitung, umfassend mindestens ein aromatisches Alkensäurederivat der Formel I 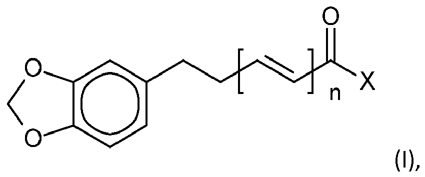 zur Anwendung in einem therapeutischen Verfahren oder in einer nicht-therapeutischen Verwendung,
(i) zur Reduzierung des Appetits,
und/oder
(ii) zur Vermittlung eines Gefühls von Sättigung,
und/oder
(iii) als Stimmungsaufheller,
wobei
n = 1, 2 bedeutet, wobei die resultierende(n) Doppelbindung(en) unabhängig voneinander als E-oder Z-Konfiguration vorliegen,
X = - OR¹ oder - NR²R³, mit
R¹= Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
und wobei
R², R³ unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
oder
R² und R³ zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2- oder -CH=CH-CH=CH-CH= .

8. Zubereitung zur Anwendung oder Verwendung nach Anspruch 7, wobei
n= 1, 2, wobei die resultierende(n) Doppelbindung(en) zu mehr als 50% in E-Konfiguration vorliegt bzw. vorliegen,
X = - OR¹ oder -NR²R³, mit
R¹= Methyl, Ethyl
R² = Wasserstoff,
R³ = 2-Methylpropyl, 2-Methyl-1-butyl,
oder
R² und R³ bilden zusammen einen gesättigten carbocyclischen Ring bestehend aus -CH2-CH2-CH2-CH2-CH2-, aus
und
wobei die aromatischen Alkensäurederivate zu mehr als 50 % in der E-Konfiguration vorliegen.

9. Zubereitung zur Anwendung oder Verwendung nach Anspruch 7 oder 8, wobei die aromatischen Alkensäurederivate ausgewählt sind aus der Gruppe bestehend aus:
5-((1,3-Benzodioxol-5-yl)-N-isobutyl-pent-2-enamid (Verbindung 1),
7-((1,3-Benzodioxol-5-yl)-N-isobutyl-hepta-2,4-dienamid (Verbindung 2),
7-((1,3-Benzodioxol-5-yl)-1-(1-piperidyl)hepta-2,4-dien-1-on (Verbindung 3),
Methyl-7-(1,3-benzodioxol-5-yl)hepta-2,4-dienoat (Verbindung 4).

10. Zubereitung zur Anwendung oder Verwendung nach einem der Ansprüche 7 bis 9, wobei die Zubereitung neben weiteren Inhaltstoffen 2E,4E-Decadiensäure-N-isoubutylamid (trans-Pellitorin) und/oder Nonivamid (N-Nonanoylvanillylamin) umfasst.

11. Zubereitung zur Anwendung oder Verwendung nach einem der Ansprüche 7 bis 10, wobei die Menge an aromatische(n) Alkensäurederivat(en) und/oder 2E,4E-Decadiensäure-N-isoubutylamid (trans-Pellitorin) und/oder Nonivamid in einer Konzentration eingesetzt wird, die nur einen unterschwelligen Aromawert in der Einsatzkonzentration zeigen.

12. Zubereitung zur Anwendung oder Verwendung nach Anspruch 11, wobei die Gesamtkonzentration der aromatischen Alkensäurederivate in der Zubereitung in einem Bereich von weniger als 20 mg/kg vorliegt, bezogen auf die Gesamtmasse der Zubereitung.

13. Zubereitung zur Anwendung oder Verwendung nach einem der Ansprüche 7 bis 12, wobei die Zubereitung ein oral konsumierbares Produkt oder Bestandteil eines oral konsumierbaren Produktes ist.

14. Zubereitung zur Anwendung oder Verwendung nach einem der Ansprüche 7 bis 13, wobei die Zubereitung ausgewählt ist aus der Gruppe bestehend aus Kosmetika, flüssige oder feste Genussmitteln, Lebensmitteln, Halbfertigwaren, Futtermitteln und Arzneimitteln.

15. Oral konsumierbares Produkt, umfassend mindestens ein aromatisches Alkensäurederivat der Formel I 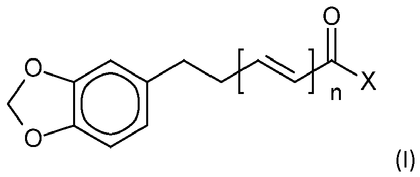 wobei
n = 1, 2 bedeutet, wobei die resultierende(n) Doppelbindung(en) unabhängig voneinander als E-oder Z-Konfiguration vorliegen,
X = - OR1 oder - NR2R3, mit
R1= Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl oder Isoprenyl,
und wobei
R2, R3 unabhängig voneinander ausgewählt ist aus der Gruppe bestehend aus: Wasserstoff, Methyl, Ethyl, Propyl, 2-Propyl, 1-Butyl, 2-Butyl, 2-Methyl-1-propyl, 1-Pentyl, 2-Pentyl, 3-Pentyl, 2-Methyl-1-butyl, 2-Methyl-2-butyl, 2-Methyl-3-butyl, 3-Methyl-1-butyl, 1-Hexyl, 2-Hexyl, 3-Hexyl, 2-Methyl-1-pentyl, 2-Methyl-2-pentyl, 2-Methyl-3-pentyl, 2-Methyl-4-pentyl, 3-Methyl-1-pentyl, 3-Methyl-2-pentyl oder 3-Methyl-3-pentyl, Allyl, Prenyl, Isoprenyl,
oder
R2 und R3 zusammen einen gesättigten oder ungesättigten carbocyclischen Ring ausbilden, ausgewählt aus der Gruppe bestehend aus: -CH2-CH2-, -CH2-CH2-CH2-, -CH2-CH2-CH2-CH2-, -CH=CH-CH=CH-, -CH2-CH2-CH2-CH2-CH2-, -CH=CH-CH2-CH2-CH2-, -CH2-CH=CH-CH2-CH2-, -CH=CH-CH=CH-CH2- oder -CH=CH-CH=CH-CH=,
wobei die Menge des/der verwendeten aromatischen Alkensäurederivate(s) in einer den Appetit reduzierenden und/ oder ein Gefühl von Sättigung bewirkenden Konzentration eingesetzt wird.
eingesetzt wird.

16. Methyl-7-(1,3-benzodioxol-5-yl)hepta-2,4-dienoat (Verbindung 4) oder ein Pflanzenextrakt, umfassend Methyl-7-(1,3-benzodioxol-5-yl)hepta-2,4-dienoat.
